# EUROPEAN PATENT SPECIFICATION

(11) **EP 0 637 963 B1**
(45) Date of publication and mention of the grant of the patent: **04.08.2004**
(21) Application number: 93910570.6
(22) Date of filing: 06.04.1993
(51) Int. Cl.: C12N 15/00, A61K 35/14, C12N 5/00, C12P 21/02, C07K 17/00

(54) **CD28 PATHWAY IMMUNOREGULATION**
IMMUNREGULATION ÜBER DIE CD28-ROUTE
IMMUNOREGULATION RECOURANT A LA VOIE D'ACCES DU CD28

(30) Priority: 07.04.1992 US 864805; 07.04.1992 US 864807; 07.04.1992 US 864866; 19.06.1992 US 902467
(43) Date of publication of application: 15.02.1995
(73) Proprietor: The Regents of the University of Michigan, Ann Arbor, MI 48109-1280 (US); The United States of America as represented by The Secretary of Navy, Bethesda, MD 20889-5606 (US)
(72) Inventor: THOMPSON, Craig, B. c/o Howard Hughes Med. Inst., M.C. 1028 Chicago IL 60637 (US); JUNE, Carl, H., Rockville, MD 20850 (US)
(74) Representative: Roques, Sarah Elizabeth
(86) International application number: PCT/US1993/003155
(87) International publication number: WO 1993/019767

(56) References cited:
- EP-A- 0 440 373
- WO-A-90/05541
- WO-A-92/00092
- PROCEEDINGS OF THE NATIONAL ACADEMY OF SCIENCES OF THE USA, vol. 86, no. 4, February 1989 WASHINGTON, DC, USA, pages 1333-1337, C. THOMPSON ET AL. 'CD28 activation pathway regulates the production of multiple T-cell-derived lymphokines/cytokines.'
- IMMUNOLOGY TODAY, vol. 11, no. 6, June 1990 AMSTERDAM, THE NETHERLANDS, pages 211-216, C. JUNE ET AL. 'Role of the CD28 receptor in T-cell activation.'
- BLOOD, vol. 75, no. 7, 1 April 1990 NEW YORK, NY, USA, pages 1531-1539, J. LEDBETTER ET AL. 'CD28 ligation in T-cell activation: Evidence for two signal transduction pathways.'
- THE FASEB JOURNAL, vol. 5, no. 5, 15 March 1991 BETHESDA, MD, USA, page A991 P. VANDENBERGHE ET AL. 'Anti-CD28 mAb 9.3 induces tyrosine phosphorylation in human T cells.'
- Eur. J. Immunology, Vol. 18, issued 1988, DARIAVACH et al., "Human Ig Superfamily CTLA-4 Gene: Chromosomal Localization and Identity of Protein Sequence Between Murine and Human CTLA-4 Cytoplasmic Domains", pages 1901-1905, see entire document.
- ROITT et al., "Immunology", published 1985, by Gower Medical Publishing (London), pages 18.2-18.3, see section under Cell-mediated Immunity to tumours - T cell responses.
- J. Immunology, Vol. 136, No. 9, issued 01 May 1986, MARTIN et al., "A 44 Kilodalton Cell Surface Homodimer Regulates Interleukin 2 Production by Activated Human T Lymphocytes", pages 3282-3287, see entire document.
- J. Immunology, Vol. 138, No. 12, issued 15 June 1987, KOZBOR et al., "Tp44 Molecules Involved in Antigen-Independent T Cell Activation are Expressed on Human Plasma Cells", pages 4128-4132, see entire document.
- J. Immunology, Vol. 135, No. 4, issued October 1985, LEDBETTER et al., "Antibodies to Tp67 and Tp44 Augment and Sustain Proliferative Responses of Activated T Cells", pages 2331-2336, see entire document.
- J. Exp. Med., Vol. 173, issued March 1991, LINSLEY et al., "Binding of the B Cell Activation Antigen B7 to CD28 Costimulates T Cell Proliferation and Interleukin 2 mRNA Accumulation", pages 721-730, see entire document.
- Proc. Natl. Acad. Sci. USA, Vol. 84, issued December 1987, ARUFFO et al., "Molecular Cloning of a CD28 cDNA by a High-Efficiency COS Cell Expression System", pages 8573-8577, see entire document.
- Proc. Natl. Acad. Sci. USA, Vol. 84, issued May 1987, GASCOIGNE et al., "Secretion of a Chimeric T-Cell Receptor-Immunoglobulin Protein", pages 2936-2940, see entire document.

## Description

### BIOLOGICAL DEPOSIT

Murine hybridoma cell line 9.3 has been deposited with the American Type Culture Collection in Rockville, MD, in compliance with the provisions of the Budapest Treaty, and has been assigned ATCC Designation No. HB10271.

### FIELD OF THE INVENTION

The present invention generally relates to immunotherapy. More particularly, the present invention relates to immunotherapy involving regulation of the CD28 T cell surface molecule.

### BACKGROUND OF THE INVENTION

Thymus derived lymphocytes, referred to as T cells, are important regulators of in vivo immune responses. T cells are involved in cytotoxicity and delayed type hypersensitivity (DTH), and provide helper functions for B lymphocyte antibody production. In addition, T cells produce a variety of lymphokines which function as immunomodulatory molecules, such as for example, interleukin-2 (IL-2), which can facilitate the cell cycle progression of T cells; tumor necrosis factor-α (TNF-α) and lymphotoxin (LT), cytokines shown to be involved in the lysis of tumor cells; interferon-γ (IFN-γ), which displays a wide variety of anti-viral and anti-tumor effects; and IL-3 and granulocyte-macrophage colony stimulating factor (GM-CSF), which function as multilineage hematopoietic factors.

Current immunotherapeutic treatments for diseases such as cancer, acquired immunodeficiency syndrome (AIDS) and attending infections, involve the systemic administration of lymphokines, such as IL-2 and IFN-γ, in an attempt to enhance the immune response. However, such treatment results in non-specific augmentation of the T cell-mediated immune response, since the lymphokines administered are not specifically directed against activated T cells proximate to the site of infection or the tumor. In addition, systemic infusions of these molecules in pharmacologic doses leads to significant toxicity. Present therapies for immunodeficient or immunodepressed patients also involve non-specific augmentation of the immune system using concentrated γ-globulin preparations. The stimulation of the *in vivo* secretion of immunomodulatory factors has not, until now, been considered a feasible alternative due to the failure to appreciate the effects and/or mechanism and attending benefits of such therapy.

It would thus be desirable to provide a method of immunotherapy which enhances the T cell-mediated immune response and which is directed specifically toward T cells activated by an antigen produced by the targeted cell. It would further be desirable to provide a method of immunotherapy which could take advantage of the patient's natural immunospecificity. It would also be desirable to provide a method of immunotherapy which can be used in immunodepressed patients. It would additionally be desirable to provide a method of immunotherapy which does not primarily rely on the administration of immunomodulatory molecules in amounts having significant toxic effects.

It would also be desirable to provide a method of immunotherapy which, if so desired, could be administered directly without removal and reintroduction of T cell populations. It would further be desirable to provide a method of immunotherapy which could be used not only to enhance, but to suppress T cell-mediated immunoresponses where such immunosuppression would be advantageous, for example, in transplant patients, in patients exhibiting shock syndrome and in certain forms of autoimmune disease.

### SUMMARY OF THE INVENTION

The present invention comprises use of CTLA4Ig in the manufacture of a medicament for use in down-regulation or suppression of the immune response for the treatment of autoimmune disease or for the treatment of patients receiving an organ transplant.

Binding of the CD28 receptor with anti-CD28 antibodies or other stimulatory binding equivalents induces activated T cell-mediated lymphokine production. Immunosuppression or down-regulation is achieved by preventing CD28 receptor binding to stimulatory ligands or inactivation of the CD28 signal transduction pathway.

We also describe the surprising and heretofore unappreciated effects of stimulation of the CD28 surface receptor molecule of activated T cells. By activated T cells is meant cells in which the immune response has been initiated or "activated," generally but not necessarily by the interaction of the T cell receptor (TCR)/CD3 T cell surface complex with a foreign antigen or its equivalent. While such activation results in T cell proliferation, it results in only limited induction of T cell effector functions such as lymphokine production.

Stimulation of the CD28 cell surface molecule with anti-CD28 antibody results in a marked increase of T cell lymphokine production. Surprisingly, even when the stimulation of the TCR/CD3 complex is maximized, upon costimulation with anti-CD28, there is a substantial increase in the levels of IL-2 lymphokine, although there is no significant increase in T cell proliferation over that induced by anti-TCR/CD3 alone. Even more surprisingly, not only are IL-2 levels significantly increased, but the levels of an entire set of lymphokines, hereinafter referred to as T_{H}CD28 lymphokines, previously not associated with CD28 stimulation are increased. Remarkably both the T cell proliferation and increased lymphokine production attributable to CD28 stimulation also exhibit resistance to immunosuppression by cyclosporine and glucocorticoids.

We also describe method by which the T cell-mediated immune response can be regulated by stimulating the CD28 T cell surface molecule to aid the body in ridding itself of infection or cancer. This can be used not only to increase T cell proliferation, if so desired, but to augment or boost the immune response by increasing the levels and production of an entire set of T cell lymphokines now known to be regulated by CD28 stimulation.

Moreover, because the effectiveness of CD28 stimulation in enhancing the T cell immune response appears to require T cell activation or some form of stimulation of the TCR/CD3 complex, the methods described herein can be used to selectively stimulate T cells preactivated by disease or treatment to protect the body against a particular infection or cancer, thereby avoiding the non-specific toxicities of the methods presently used to augment immune function. In addition, the methods described herein enhances T cell-mediated immune functions even under immunosuppressed conditions, thus being of particular benefit to individuals suffering from immunodeficiencies such as AIDS.

It will also be appreciated that although the following discussion of the principles of the present invention exemplifies the present invention in terms of human therapy, the methods described herein are similarly useful in veterinary applications.

A better understanding of the present invention and its advantages will be had from a reading of the detailed description of the preferred embodiments taken in conjunction with the drawings and specific examples set forth below.

### BRIEF DESCRIPTION OF THE DRAWINGS

Figure 1 is a bar graph illustrating the absence of augmentation of the uptake of thymidine by CD28 stimulated T cells.
Figure 2 is a bar graph illustrating the increase in uridine incorporation by CD28 stimulation of anti-CD3 stimulated T cells.
Figure 3 is a graph illustrating the elevated cyclosporine resistance of T cell proliferation induced by CD28 stimulation.
Figure 4 is a Northern blot analysis of the effects of cyclosporine on PMA-or anti-CD3 activated T cell lymphokine expression induced by anti-CD28.
Figure 5 is a graph illustrating *in vivo* activation of T cells in monkeys by CD28 stimulation.
Figures 6A and 6B are graphs representing changes in lymphocyte levels after infusion of anti-CD28 mAb.
Figures 7A and 7B are graphs representing *in vitro* production of TNF and IL-6 by PBLs under various conditions.
Figures 8A and 8B are graphs representing serum concentration of IL-1β after single and multiple doses of anti-CD28 mAb.
Figures 9A and 9B are graphs representing the serum concentration of IL-6 after single and multiple doses of anti-CD28 mAb.
Figures 10A and 10B are graphs representing IL-6 production of *in vitro* stimulated PBLs isolated from monkeys treated with a single bolus or multiple injections of anti-CD28 mAb.
Figure 11 is a graph representing the inhibitory effect of CTLA4Ig on ³H-thymidine incorporation in a one-way mixed lymphocyte culture.
Figures 12A and 12B are photographs of cardiac allografts to illustrate histopathology.
Figure 13 is a Kaplan-Meier life analysis of cardiac allograft survival after CTLA4IgG treatment.
Figure 14 is a bar graph illustrating CTLA4Ig and cyclosporine as synergistic immunosuppressants.
Figure 15 is a bar graph illustrating the effect of herbimycin A on CD28-stimulated IL-2 production.
Figure 16 is a bar graph illustrating activation by SEB and anti-CD28 on purified resting T cells in the presence and absence of a blocking mAb to HLA-DR.
Figure 17 is a bar graph illustrating activation by SEB alone or SEB and blocking mAb to HLA-DR in peripheral blood mononuclear cells.
Figure 18 is a graph showing *in vitro* long term growth of CD4⁺ peripheral blood T cells propagated with anti-CD3 and anti-CD28.
Figure 19 is a Northern blot analysis of the enhancement of mRNA for IL-2 and TNF-α after costimulation with anti-CD3 and anti-CD28.
Figure 20 is a Northern blot analysis of the ability of mitogens to induce CTLA-4 mRNA expression.
Figure 21 is a Northern blot analysis of the induction of CTLA-4 mRNA expression by costimulation with anti-CD3 mAb and soluble anti-CD28 mAb.

### DETAILED DESCRIPTION OF THE PREFERRED EMBODIMENTS

In one embodiment described herein , the CD28 molecule is stimulated to enhance the T cell-mediated immune response of antigen or otherwise activated T cells. CD28 is a 44 kilodalton protein expressed on the surface of about 80% mature T cells which exhibits substantial homology to immunoglobulin genes. See Poggi, A. et al., *Eur*. *J. Immunol*., 17:1065-1068 (1987) and Aruffo, A. et al., *PNAS (USA)*, 84:8573-8577 (1987). Binding of the CD28 molecule's extracellular domain with anti-CD28 antibodies in accordance with the method of the present invention results in an increase in T cell proliferation and elevated lymphokine levels.

In Specific Examples III-IV and VI-VIII, T cell activation was accomplished by stimulating the T cell TCR/CD3 complex (which mediates the specificity of the T cell immune response) with immobilized anti-CD3 monoclonal antibodies, such as mAb G19-4, or by chemically stimulating with PMA and ionomycin. It should also be appreciated, however, that activation of the T cell can instead be accomplished by routes that do not directly involve CD3 stimulation, such as the stimulation of the CD2 surface protein.

In practice, however, an activated T cell population will be provided by the patient's own immune system, which, barring total immunosuppression, will have T cells activated in response to any foreign or substantially elevated level of antigen present due to disease, infection, inoculation or autoimmunity. The term "foreign antigen" is used broadly herein, meaning an antigen which is either not normally produced by the organism, or, as in carcinomas, an antigen which is not normally produced by the cell which is producing it. The term is also meant to include an antigen which should normally be seen as "self," but, as occurs in autoimmune disease states, provokes an immune response as would a foreign antigen. By "substantially elevated" level of antigen is meant an antigen level exceeding normal ranges and having potentially deleterious effects to the organism due to such elevation.

Stimulation of the CD28 molecule itself may be achieved by administration of a ligand, such as a monoclonal antibody or a portion thereof, (*e*.*g*. F(ab')₂), having a binding specificity for and stimulatory effect on CD28. Suitable antibodies include mAb 9.3, an IgG2a antibody on deposit with the ATCC which has been widely distributed and is available (for non-commercial purposes) upon request from Dr. Jeffrey A. Ledbetter of Oncogen Corporation, Seattle, WA, or mAb KOLT-2. Both these monoclonal antibodies have been shown to have binding specificity for the extracellular domain of CD28 as described in "Leukocyte Typing II," Ch. 12, pgs. 147-156, ed. Reinhertz, E. L et al. (1986). Monoclonal antibody 9.3 F(ab')₂ has also been shown to be a satisfactory ligand capable of stimulating the CD28 receptor. The use of chimeric antibodies as well as non-immunoglobulin, natural and recombinant ligands which bind the CD28 surface molecule is also contemplatad More recently, the natural ligand for CD28, B7/BB1, has also been identified and can be used in accordance with the principles of the present invention. See Linsley, P.S. et al., *J. Exp. Med*. 174:561 (1991). In addition, binding homologs of a natural ligand, whether natural or synthesized by biochemical, immunological, recombinant or other techniques, can also be used. It will be appreciated that the ligands referred to herein can be utilized in their soluble or cell-bound forms, depending on their application. Monoclonal antibody 9.3 and B7 are currently preferred stimulatory ligands.

The extracellular domain of CD28, which was sequenced by Aruffo, A. et al., *PNAS (USA)*, 84:8573-8577 (1987), generally comprises the following amino acid, sequence: 
MetLeuArgLeuLeuLeuAlaLeuAsnLeuPheProSerIleGln
ValThrGlyAsnLysIleLeuValLysGlnSerProMetLeuVal
AlaTyrAspAsnAlaValAsnLeuSerCysLysTyrSerTyrAsn
LeuPheSerArgGluPheArgAlaSerLeuHisLysGlyLeuAsp
SerAlaValGluValCysValValTyrGlyAsnTyrSerGlnGln
LeuGlnValTyrSerLysThrGlyPheAsnCysAspGlyLysLeu
GlyAsnGluSerValThrPheTyrLeuGlnAsnLeuTyrValAsn
GlnThrAspIleTyrPheCysLysIleGluValMetTyrProPro
ProTyrLeuAspAsnGluLysSerAsnGlyThrIleIleHisVal
LysGlyLysHisLeuCysProSerProLeuPheProGlyProSer
LysPro
 By the term "extracellular domain" as used hereinafter in the specification and claims, is meant the amino acid sequence set forth above, any substantial portion thereof, or any sequence having substantial homology thereto.

As shown by the data of Specific Examples III-V, substantial augmentation of the T cell-mediated immunoresponse by CD28 stimulation appears specific for activated T cells. Such specificity is of particular clinical importance and is one of the significant advantages of the method of immunotherapy described herein. Administration of anti-CD28 antibodies or other CD28 ligands will specifically augment the response of T cells which are already activated and engaged in the immune response or those in the process of activation. It should, however, also be appreciated that CD28 stimulation may be effective even where the T cells are activated after the binding of the CD28-specific ligand to CD28 receptor. Thus, the T cells at or near the tumor site or site of infection, which are being activated by the antigens produced or present at those sites, will be selectively "boosted" by the CD28 stimulation.

Boosting of the immune response can also be beneficial to healthy individuals, for example, in augmenting their response to antigens presented in vaccines (see Specific Example IX). CD28 stimulation coupled with antigen administration in accordance with the present invention can result in more effective immunization, not only with conventional vaccines, but in situations where an adequate immune response is difficult to elicit, e.g. with human retroviruses such as HIV and some herpes viruses. Examples where CD28 stimulation can be used include, but are not limited to viral vaccines against measles, influenza, polio. herpes (*i*.*e*. HCMV, Epstein Barr Virus, Type I and II); bacterial vaccines against whooping cough (*Bordatella pertussis*), tetanus (*Clostridium tetanus*), pneumonia (*Streptococcus pneumoniae*), meningitis and gonorrhea *(Neisseria)* and against enteropathic bacteria such as *Salmonella, E. coli* and *Shigella*. These principles are also applicable in inoculations against parasitic infection, including those caused by protozoal parasites, e.g. malaria, trypanosomiasis, leishmaniasis, amebiasis, toxoplasmosis, pneumocystis carinni and babesiosis, by cestodes (*e*.*g*. tapeworm) and by other parasitic organisms. It will also be appreciated that immunization for a humoral response through injection of cDNA for intracellular antigenic production, as described in *Nature* 356:152 (1992), costimulated with anti-CD28 is also contemplated.

When TCR/CD3 stimulation is maximized, although T cell proliferation is not markedly increased, the levels of certain lymphokines are substantially increased by CD28 activation, indicating an increase in cellular production of these lymphokines. Thus, in patients undergoing natural maximal TCR/CD3 stimulation or its equivalent T cell activation *in vivo* due to disease or infection, the administration of anti-CD28 antibody or other CD28 ligand to stimulate CD28 will result in substantially elevated lymphokine production.

The increase in lymphokine production achieved by administration of a CD28 stimulator as particularly shown in Specific Example III, surprisingly results in the increased production of an entire set of lymphokines, indicating that these lymphokines are under some form of CD28 regulation. Part of this set of lymphokines, which includes IL-2, TNF-α, LT, IFN-γ, and IL-3 as later determined, is somewhat analogous to the T_{H}1 cell lymphokines present in the mouse which were described by Mosmann, T. R. et al., *Immunol. Today*, 8:223-227 (1987). Although it was originally believed that human IL-4 production was not increased by CD28 stimulation, more recent assays as set forth in Specific Example III have now also shown an increase in the production of other lymphokines, including IL-4 and IL-5 and the increased production of IL-6 and IL-1 was also confirmed in Specific Example IX. It will be appreciated, however, that the term "T_{H}1 lymphokines" was originally used for ease of reference and was expressly not limited to the lymphokines listed above, but was meant to include all lymphokines whose production is affected or regulated by the binding or stimulation of the CD28 T cell surface molecule. Thus the group of lymphokines affected by CD28 will hereinafter be referred to as T_{H}CD28 lymphokines, it will again be appreciated that the term T_{H}CD28 lymphokine is not intended to be limiting to the specific lymphokines listed herein. Furthermore, it will be appreciated that these principles can be used in veterinary applications to increase the T cell-mediated immune response and lymphokine production in animals. The term T_{H}CD28 lymphokines, as used herein, is thus also meant to include analogous animal lymphokines. Thus, by administration of anti-CD28 antibodies or other CD28 ligands , the production and levels of an entire set of lymphokines can be significantly increased.

The method described herein can also be used to facilitate the T cell-mediated immune response in immunodepressed patients, such as those suffering from AIDS. As shown in Specific Examples VI - VIII, T cell proliferation and the increased levels or production of CD28-regulated lymphokines continue to function even in the presence of immunosuppression such as that caused by cyclosporine or dexamethasone. Thus administration of CD28 stimulators such as mAb 9.3 or other CD28 ligands can be used to treat immunodepressed patients to increase their *in vivo* lymphokine levels.

In addition, a variety of syndromes including septic shock and tumor-induced cachexia may involve activation of the CD28 pathway and augmented production of potentially toxic levels of lymphokines. The immune response can also be deleterious in other situations such as in organ transplant recipients or in autoimmune disease. Thus down-regulation or inactivation of the CD28 pathway, as discussed more fully below and in Specific Examples X and XI, can also provide immunotherapy for those and other clinical conditions.

It should be appreciated that administration of an anti-CD28 antibody has not heretofore been seriously contemplated as a potential immunotherapeutic method for the substantial increase of lymphokine levels at the sites of activated T cells. For example, the addition of mAb 9.3 has been thought only to somewhat augment T cell proliferation, not to induce substantial increases in T_{H}CD28 lymphokine production.

Although it is not the intent herein to be bound by any particular mechanism by which CD28 binding regulates the T cell-mediated immune response, a model for the mechanism of stimulation has been postulated and supported with experimental data, some of which is shown in Specific Example VIII. It has previously been shown that a number of lymphokine genes undergo more rapid degradation in the cytoplasm than mRNAs from constitutively expressed housekeeping genes, leading to the hypothesis that the instability of these inducible mRNAs has been selected to allow for rapid regulation of gene expression. It is believed that the mechanism of CD28 regulation herein described and claimed is related to the stabilization of rapidly degradable mRNAs for the set of T_{H}CD28 lymphokines set forth above. To date, it appears no other mechanism in any eukaryotic cell system has been described to demonstrate that a cell surface activation pathway can alter gene expression by inducing specific alteration in mRNA degradation. (A more in-depth analysis of possible models of CD28 activation is presented later herein.)

As shown in Specific Example IV, costimulation of CD28 and TCR/CD3 caused an increase in mRNA of the T_{H}CD28 lymphokines which was not the result of a generalized increase in a steady state mRNA expression of all T cell activation-associated genes. The increase was disproportionate and thus could not be accounted for by the increase in percentage of proliferating cells in culture. These data, in addition to further studies not detailed herein, demonstrate that activation of the CD28 surface molecule of activated T cells functions to specifically stabilize lymphokine mRNAs. Increased mRNA stability, *i*.*e*. slower degradation thereof, results in increased translation of the mRNA, in turn resulting in increased lymphokine production per cell. An increase in per T cell production of lymphokines that allows the T cell to influence the response of other inflammatory and hematopoietic cells is referred to as paracrine production. In contrast, an increase in lymphokine levels merely due to increased cell proliferation, such as that shown in Martin, P.J. et al., J. *Immunol*. 136:3282-3287 (1986), is commonly referred to as autocrine production. For ease of reference, paracrine production is also herein referred to as "cellular" production of lymphokines.

Thus, ligands with binding specificity for the CD28 molecule are administered in a biologically compatible form suitable for administration *in vivo* to stimulate the CD28 pathway. By "stimulation of the CD28 pathway" is meant the stimulation of the CD28 molecule resulting in increased T cell production of T_{H}CD28 lymphokines. By "biologically compatible form suitable for administration *in vivo*" is meant a form of the ligand to be administered in which the toxic effects, if any, are outweighed by the therapeutic effects of the ligand. Administration of the CD28 ligand can be in any suitable pharmacological form, which includes but is not limited to intravenous injection of the ligand in solution.

It should be understood that, although the models for CD28 regulation of lymphokine production are described with respect to stimulation and enhancement of lymphokine levels, as noted above, down-regulation or inhibition of the CD28 pathway is also in accordance with the principles of the present invention. Down-regulation or suppression of the immune response is of particular clinical interest for a variety of conditions, including septic shock, tumor-induced cachexia, autoimmune diseases and for patients receiving heart, lung, kidney, pancreas, liver and other organ transplants. One preferred approach to down-regulation is the blocking of the CD28 receptor stimulatory binding site on its natural ligand. For example, CTLA-4 (discussed in more detail below), which shares 32% amino acid homology with CD28 and appears to have greater binding affinity for B7 than CD28, can be used to bind B7 and prevent CD28 binding and activation thereby. See Linsley, P.S. et al., *J. Exp. Med*., 174:561 (1991). Such regulation has been accomplished *in vivo* as described in Specific Example X. In this Example, acute rejection of fully MHC-mismatched cardiac allografts was prevented by blocking B7-dependent T cell activation, *i*.*e*. CD28 binding, with the soluble recombinant fusion protein CTLA4Ig. The immunosuppression observed with CTLA4Ig did not result in significant alterations in circulating T cell subsets. It will be appreciated that other B7-binding ligands such as a monoclonal antibody to B7 can be similarly employed.

It will be appreciated that down-regulation can also be accomplished by blocking CD28 receptor binding to B7 by occupying the CD28 binding site with nonstimulatory ligands which may mimic stimulatory ligands but do not result in activation of the CD28 pathway, e.g. F(ab')s, modified natural, synthetic, recombinant or other ligands which do not crosslink or otherwise do not activate receptors.

Accumulating evidence suggests that in addition to T cell receptor occupancy, other costimulatory signals are required to induce a complete T cell-mediated immune response. The CD28 receptor expressed on T cells serves as a surface component of a novel signal transduction pathway that can induce paracrine levels of cellular production of lymphokines. *In vitro*, interaction of CD28 with its natural ligand 87 which is expressed on the surface of activated B cells macrophages or dendritic cells can act as a costimulus to induce high level lymphokine production in antigen receptor-activated T cells. Thus, another approach to down-regulation is to inhibit the activation of the CD28 signal transduction pathway as described below.

Although the CD28 signal transduction pathway is not entirely understood, Specific Example XI demonstrates that binding of CD28 induces protein tyrosine phosphorylation distinct from T cell receptor (TCR)-induced tyrosine phosphorylation. For example, TCR-induced tyrosine phosphorylation occurs in both resting and activated T cells, while CD28-induced tyrosine phosphorylation occurs primarily in previously activated T cells. Most striking were the results after CD28 receptor ligation by cell-bound B7, where phosphorylation was consistently detectable on only a single substrate. Experiments using the Jurkat E6-1 T cell line indicated an absolute requirement for PMA pretreatment in order to observe CD28-induced tyrosine phosphorylation. In contrast, there was no requirement for cellular preactivation in the Jurkat J32 line, while, as noted above, there was a relative requirement for PMA or TCR prestimulation of normal T cells in order to induce CD28 responsiveness. Studies with Jurkat mutants further indicated that CD28-induced tyrosine phosphorylation and biologic function can occur in the absence of the TCR. In this respect, CD28 appears to be unique in that other accessory molecules involved in T cell activation, such as CD2, Ly-6, Thy-1, and CD5 appear to require the presence of the TCR. Thus, specific tyrosine phosphorylation appears to occur directly as a result of CD28 ligand binding and is involved in transducing the signal delivered through CD28 by accessory cells that express the B7/BB1 receptor.

Studies with an inhibitor of the src family of tyrosine kinases, herbimycin A, and with tyrosine phosphatase, as described in Specific Example XI, futher show that the functional effects of CD28 stimulation on lymphokine gene expression require the above-described protein tyrosine phosphorylation. The data on tyrosine phosphorylation inhibitors thus demonstrate that inactivation of CD28- mediated signal transduction can also be used to down-regulate lymphokine production in accordance with the principles of the present invention.

Immunotherapy through CD28 stimulation also has clinical applicability in the treatment of bone marrow transplant recipients. The success of autologous and allogeneic bone marrow transplantation (BMT) has generally been limited by recurrent malignancy, graft-vs-host disease (GVHD), and the life-threatening immune deficiency that occurs after BMT. One approach to overcoming these problems has been the adoptive transfer of lymphocytes in combination with lymphokine infusions, to accelerate immune reconstitution or mediate cytotoxicity directed at malignant cells. The side effects attending such transfer with lymphokine infusions are, however, quite significant.

Thus, T cell proliferation and lymphokine synthesis in the absence of exogenously added IL-2 in response to CD3 and CD28 costimulation, as described in Specific Example XIII, provides a unique opportunity for clinical use of adoptive transfer of activated T cells to repair T cell defect *in vivo* without exogenous lymphokine infusions. The studies detailed in Specific Example XIV show that defective *in vitro* proliferative responses to anti-CD3 (OKT3 or G19-4) can be repaired by adding mAb 9.3 to the cultures. See Joshi, I. et al., *Blood Suppl*. (Abstract) (1991). Costimulation of T cells with OKT3/9.3 repaired proliferative responses as a result of increasing the levels of mRNA expression for cytokines/lymphokines such as IL-2, GM-CSF, and TNFα. See Joshi, *supra;* Perrin, P.J. et al, *Blood Suppl.* (Abstract) (1991). Purified normal CD4⁺ cells can thus be costimulated with OKT3/9.3 to expand and secrete lymphokines for long periods of time. Preclinical studies using mAb 9.3 stimulation have shown no untoward effects in monkeys. Although CD3-stimulated cells (CTC) provide helper factors to normal B cells, CD3-CD28 costimulated cells appear even more effective in producing helper factors than CD3-stimulated T cells. Thus, costimulation may also enhance the growth of helper cells and cytotoxic T cells for adoptive immunotherapy after BMT. The administration *in vivo* of T cells that have been expanded *in vitro,* will provide two prominent benefits in marrow transplantation. The abillity of CD28- treated cells to produce many lymphokines which have a strong positive effect on hematopoiesis, such as GM-CSF, IL-3, and IL-6, should accelerate engraftment after marrow transplantation. In addition, the abililty of anti-CD28 to trigger cytotoxicity and to cause the production of lymphokines such as TNF is a novel form of adoptive immunotherapy that should augment the anti-neoplastic efficacy of bone marrow transplantation.

The possible role of CD28 in anergy was also examined. Generally, the activation of a quiescent T cell is initiated through stimulation of the T cell antigen receptor. This activation can occur either through engagement of an antigenic peptide presented in the antigen binding groove of a self-encoded MHC molecule or by engagement of a foreign MHC molecule. However, while this receptor-mediated activation event is required for the initiation of a T cell response in a quiescent cell, recent studies have demonstrated that signals transduced by the antigen receptor alone are not sufficient to lead to an effective T cell-mediated immune response. Several *in vitro* models suggest that, in fact, T cell receptor (TCR)/CD3 activation alone of a quiescent T cell leads to the induction of a state in which the T cell becomes anergic to further stimulations through its antigen-specific receptor. This state is relatively long-lived and, for at least several weeks, renders that cell incapable of further response upon antigenic stimulation. It is hypothesized that this isolated activation of the TCR/CD3 complex alone in the absence of additional T cell costimulatory molecules plays an important role in regulating a peripheral immune response by preventing T cells from responding to self antigens in the periphery.

Thus, for T cells to mount a proliferative response and initiate a cell-mediated immune response, a quiescent T cell normally requires stimulation not only through its antigen-specific T cell receptor but also through a second receptor which provides additional costimulatory signals to the cell. The data set forth herein, *e*.*g*. in Specific Example XII, demonstrates that CD28 provides an essential costimulatory signal for T cell responses *in vitro* and *in vivo.* Thus, the CD28 receptor's ability to augment T cell lymphokine production not only results in the initiation of a cell-mediated immune response, but also prevents the induction of anergy in a quiescent T cell.

The role of CD28 in the prevention of programmed cell death has also been tested. The induction of cell death has a major role in the elimination of self-reactive or non-reactive T cells in the thymus. In the thymus, it is thought that T cell receptor signals are able to induce programmed cell death, in a selective and specific fashion involving cells that express T cell receptors specific for self-antigens. As described in Turka, L.A. et al., *J. Immunol*., 144:1646-53 (1990), CD28 is expressed in developing T cells in the thymus, and the binding of mAb 9.3 prevents thymocyte cell death. Programmed cell death is also thought to occur in mature T cells in peripheral lymphoid organs. Signals delivered through the T cell receptor can induce cell death (see Newell, M.K., et al., *Nature*, 347:286-8 (1990)). It has also been proposed that cell death may have a role in certain forms of immunopathology. For example, in HIV-1 infection it has been proposed that the progressive immunodeficiency may be the result of immunologically-mediated cell death, rather than a direct consequence of viral-induced cytopathic effects. See Ameisen, J.C. et al., *Immunol. Today,* 4:102 (1991); also see Groux H., et al., *J. Exp. Med*. 175:331-340 (1992). The results in Specific Example XIII show that CD28 can prevent cell death in mature T cells. Thus, abnormal expression or activation of CD28 may have a role in immunopathology of certain autoimmune disorders such as systemic lupus erythematosus, a disorder characterized by abnormally self-reactive T cells that have failed to undergo elimination in the thymus or escape from anergic states in the peripheral lymphoid system. Similarly, the ability to induce CD28 activation may be beneficial in disorders characterized by progressive T cell depletion such as HIV-1 infection.

It was also demonstrated in Specific Example XII that superantigens SEA and SEB, which do not require traditional processing for binding to MHC, can directly activate purified T cells in the absence of accessory cells as determined by a transition from G₀ to G₁ and induction of IL-2 receptor expression. However, neither SEA nor SEB alone was sufficient to result in T cell proliferation. The induction of T cell proliferation by SEB or SEA required the addition of a second costimulatory signal. This could be provided by either accessory cells or monoclonal antibody stimulation of CD28. As previously reported, T cell proliferation induced by enterotoxin in the presence of accessory cells was partially inhibited by a blocking antibody (HLA-DR) against class II MHC. In contrast, in purified T cells when costimulation was provided through CD28, proliferation was not inhibited by class II antibody and HLA-DR expression was not detectable. In addition, costimulation through CD28 was partially resistant to the effects of cyclosporine. These results demonstrate that CD28 costimulation is sufficient to induce lymphokine production and subsequent proliferation of enterotoxin-activated T cells, and that this effect is independent of class II MHC expression. This prevention of *in vivo* CD28 activation of superantigen-activated cells such as those occurring in toxic shock syndrome and rheumatoid or lyme arthritis, may substantially decrease disease morbidity.

Although as noted previously the present invention is not intended to be restricted to specific mechanisms of activation, the role of CTLA-4 in the CD28 activation pathway has been examined and models consistent with the data presented herein have been postulated. (See e.g. Specific Example XV.) Recent work in our laboratory has shown that the CTLA-4 gene lies immediately adjacent to CD28 on chromosome 2, with a similar genomic organization and 32% amino acid homology. Based on their chromosomal localization and sequence and organizational similarities, CD28 and CTLA-4 likely represent evolutionary gene duplication. By standard nomenclature they might thus more appropriately be named CD28α and CD28β, although the terms CD28 and CTLA-4 are retained herein.

Although CTLA-4 is not expressed on quiescent lymphoid cells, its expression at the RNA level can be rapidly induced upon T cell activation. Two potential mechanisms by which CTLA-4 might function are postulated as follows. First, since CD28 and CTLA-4 contain an unpaired cysteine in their extracellular domain, this cysteine residue may be used to form crosslinked dimeric receptors on the surface. If this were the case, it may suggest that CTLA-4 is normally expressed on the surface as a heterodimer with CD28. Under such conditions, the higher affinity of CTLA-4 for the natural ligand B7 might in the dimeric state lead to a higher affinity receptor with enhanced signaling capabilities. This might allow for an enhanced signal transduction capability through the CD28-CTLA-4 heterodimer in an antigen-activated cell. In addition, if CD28 and CTLA-4 are found primarily in activated cells in a hoterodimeric state, this might account for observations that CD28-containing receptors have enhanced signaling capabilities in activated cells.

On the other hand, the data presented herein are also compatible with a model in which CTLA-4 is induced upon T cell activation as a competitive inhibitor of CD28 and is used to down-modulate an ongoing immune response by inhibiting further interactions between B7 and CD28 on the surface. In addition, it is quite possible that the CTLA-4 expressed on the surface is also expressed in a shed form, and this shed form of the receptor acts as a soluble competitive inhibitor of an ongoing B7-CD28 interaction, thereby preventing the antigen-presenting cell from activating additional T cells in its environment. Thus, the ability of T cells to produce an additional isoform of CD28, *i*.*e*. CTLA-4, suggests that the interplay of expression of CD28 and CTLA-4 has profound effects on the ability of T cells to be activated through a CD28-containing receptor.

CD28 pathway activation and inhibition studies indicate that the abililty of the CD28 natural ligand B7 to activate a T cell to augment lymphokine production is entirely mediated through a CD28-containing receptor, either a CD28 homodimer or a CD28-CTLA-4 heterodimer. Thus, the data suggest that a CTLA-4 homodimer is not critical in T cell activation, but may play an important role in down-modulation of T cell lymphokine production, while a CD28-CTLA-4 heterodimer may account for the enhanced signaling properties of CD28-containing receptors upon T cell activation.

The role of CTLA-4 in CD28-mediated signal transduction event may explain why the novel and profound effects of CD28 on normal T cell activation encountered and described herein were not previously observed in human T cell lines. Earlier work on the CD28 pathway occurred in cell lines such as Jurkat human T cells. Extensive attempts to stimulate these cells to express CTLA-4 have been entirely negative (see Figure 20). In contrast, standard activation events that lead to cell cycle progression of normal T cells either through chemical mitogens such as phytohemagglutinin (PHA) and phorbol myristate acetate (PMA) leads to rapid induction of CTLA-4 expression as does crosslinking of the TCR/CD3 complex. Therefore, the inability of previous investigators to appreciate or harness the CD28 activation pathway to enhance cellular production of lymphokines was likely due to the lack of expression of the CTLA-4 isoform of CD28 in these cell lines. Interestingly, costimulation of resting T cells with anti-CD28 monoclonal antibodies enhances the expression of the CTLA-4 gene (see Figure 21). Thus, the CD28 activation pathway in normal cells may in fact involve a positive feedback loop in which initial CD28 stimulation through the CD28 homodimer enhances the expression of CTLA-4 thus leading to enhanced heterodimer expression and signal transduction. Alternatively, the enhanced CTLA-4 may lead to the production of a receptor which competes for CD28 signal transduction thus leading to the ultimate termination of lymphokine production and acts as a negative feedback loop to down modulate an ongoing CD28-mediated lymphokine production.

### SPECIFIC EXAMPLE I

### Preparation of CD28 Stimulator Monoclonal Antibody 9.3.

The monoclonal antibody (mAb) 9.3, an IgG2a monoclonal antibody which binds to the extracellular domain of the CD28 molecule, was produced by a hybrid cell line originally derived as described by Hansen et al., *Immunogen*., 10:247-260 (1980). Ascites fluid containing high titer monoclonal antibody 9.3 was prepared by intraperitoneal inoculation of 5 - 10 x 10⁶ hybrid cells into a Balb/C x C57BL/6 F₁ mice which had been primed intraperitoneally with 0.5 ml of Pristane (Aldrich Chemical Co., Milwaukee, WI). The monoclonal antibody 9.3 was purified from ascites fluid on a staphylococcal protein-A sepharose column as described by Hardy, R., "Handbook of Experimental Immunology," Ch. 13 (1986).

Prior to use in functional assays, purified mAb 9.3 was dialyzed extensively against phosphate buffered saline (KCI 0.2 grams/liter dH₂O; KH₂PO₄ 0.2 grams/liter dH₂O; NaCl 8.0 grams/liter dH₂O; Na₂HPO₄·7H₂O 2.16 grams/liter dH₂O) and then filtered through a 0.22 cubic micron sterile filter (Acrodisc, Gelman Sciences, Ann Arbor, MI). The mAb 9.3 preparation was cleared of aggregates by centrifugation at 100,000 xg for 45 m at 20°C. The resulting purified mAb 9.3 was resuspended in phosphate buffered saline to a final concentration of 200 µg/ml as determined by OD280 analysis and stored at 4°C prior to use.

### SPECIFIC EXAMPLE II

### Isolation of CD28⁺ T Cells.

Buffy coats were obtained by leukopheresis of healthy donors 21 to 31 years of age. Peripheral blood lymphocytes (PBL), approximately 2.5 x 10⁹, were isolated from the buffy coat by Lymphocyte Separation Medium (Litton Bionetics, Kensington, MD) density gradient centrifugation. The CD28⁺ subset of T cells was then isolated from the PBL by negative selection using immunoabsorption, taking advantage of the reciprocal and non-overlapping distribution of the CD11 and CD28 surface antigens as described by Yamada et al., *Eur*. *J. Immunol*., 15:1164-1688 (1985). PBL were suspended at approximately 20 x 10⁶/ml in RPMI 1640 medium (GIBCO Laboratories, Grand Island, NY) containing 20mM HEPES buffer (pH 7.4) (GIBCO Laboratories, Grand Island, NY), 5mM EDTA (SIGMA Chemical Co., St. Louis, MO) and 5% heat-activated human AB serum (Pel-Freez, Brown Deer, WI). The cells were incubated at 4°C on a rotator with saturating amounts of monoclonal antibodies 60.1 (anti-CD11a) (see Bernstein, I.D. et al., "Leukocyte Typing II," Vol. 3, pgs. 1-25, ed. Reinherz, E. L. et al., (19B6); 1F5 (anti-CD20) (see Clark, E. A. et al., *PNAS(USA),* 82:1766-1770 (1985)); FC-2 (anti-CD16) (see June, C. H. et al., *J. Clin. Invest*., 77: 1224-1232 (1986)); and anti-CD14 for 20 m. This mixture of antibodies coated all B cells, monocytes, large granular lymphocytes and CD28⁻ T cells with mouse immunoglobulin. The cells were washed three times with PBS to remove unbound antibody, and then incubated for 1 h at 4°C with goat anti-mouse immunoglobulin-coated magnetic particles (Dynal, Inc., Fort Lee, NJ) at a ratio of 3 magnetic particles per cell. Antibody-coated cells that were bound to magnetic particles were then removed by magnetic separation as described by Lea, T. et al., Scan. *J. Immunol*., 22:207-216 (1985). Typically, approximately 700 x 10⁶ CD28⁺ T cells were recovered. Cell purification was routinely monitored by flow cytometry and histochemistry. Flow cytometry was performed as described by Ledbetter, J. A. et al., "Lymphocyte Surface Antigens," pgs. 119-129 (ed. Heise, E., 1984). Briefly, CD28⁺ T cells were stained with fluorescein isothiocyanate (FITC)-conjugated anti-CD2 mAb OKT11 (Coulter, Hialeah, FL) and with FITC-conjugated anti-CD28 mAb 9.3 as described by Goding, J. W., "Monoclonal Antibodies Principles and Practice," p. 230 (ed. Goding, J. W., 1983). CD28⁺ T cells were over 99% positive with FITC-conjugated monoclonal antibody OKT11 and over 98% positive FITC-conjugated monoclonal antibody 9.3 when compared to a non-binding, isotype-matched, FITC-labeled control antibody (Coulter, Hialeah, FL). Residual monocytes were quantitated by staining for non-specific esterase using a commercially available kit obtained from Sigma Chemical Co., St. Louis, MO, and were less than 0.1% in all cell populations used in this study. Viability was approximately 98% as measured by trypan blue exclusion as described by Mishell, B.B. et al., "Selected Methods in Cell. Immunology," pgs.16-17 (1980).

### SPECIFIC EXAMPLE III

### Increased Cellular Production of Human T_{H}CD28 Lymphokines by CD28 Stimulation by Monoclonal Antibody 9.3.

### A. Increased Production of IL-2, TNF-α, IFN-γ and GM-CSF.

CD28⁺ T cells were cultured at approximately 1 x 10⁵ cells/well in the presence of various combinations of stimulators. The stimulators included phorbol myristate acetate (PMA) (LC Services Corporation, Woburn, MA) at 3 ng/ml conc.; anti-CD28 mAb 9.3 at 100 ng/ml; anti-CD3 mAb G19-4 at 200 ng/ml which was immobilized by adsorbing to the surface of plastic tissue culture plates as previously described by Geppert, et al., *J*. *Immunol*., 138:1660-1666 (1987); also Ledbetter, et al., *J. Immunol*., 135: 2331-2336 (1985); ionomycin (Iono) (Calbiochem., San Diego, CA) at 100 ng/ml. Culture supernatants were harvested at 24 h and serial dilutions assayed for the presence of T_{H}CD28 lymphokines.

Specifically, IL-2 was assayed using a bioassay as previously described by Gillis et al., *Nature,* 268:154-156 (1977). One unit (U) was defined as the amount of IL-2 needed to induce half maximal proliferation of 7 x 10³ CTLL-2 (a human cytotoxic T cell line) cells at 24 h of culture. In separate experiments, the relative levels of IL-2 for each of the culture conditions above were independently confirmed using a commercially available EUSA assay (Genzyme Corp., Boston, MA). TNF-α/LT levels were measured using a semi-automated L929 fibroblast lytic assay as previously described by Kunkel et al., *J. Biol. Chem*., 263:5380-5384 (1988). Units of TNF-α/LT were defined using an internal standard for TNF-α (Genzyme Corp., Boston MA). The independent presence of both TNF-α and LT was confirmed by the ability of a monoclonal antibody specific for each cytokine to partially inhibit cell lysis mediated by the supernatant from cells costimulated with immobilized anti-CD3 mAb G19-4 and anti-CD28 mAb 9.3. IFN-γ was measured by radioimmunoassay using a commercially available kit (Centocor, Mahrern, PA). Units for IFN-γ were determined from a standard curve using ¹²⁵I-labeled human IFN-γ provided in the test kit. GM-CSF was detected by stimulation of proliferation of the human GM-CSF-dependent cell line AML-193, as described by Lange et al., Blood, 70:192-199 (1987), in the presence of neutralizing monoclonal antibodies to TNF-α and LT. The ³H-thymidine uptake induced by 10 ng/ml of purified GM-CSF (Genetics Institute, Cambridge, MA) was defined as 100 U. Separate aliquots of cells were recovered 48 h after stimulation and assayed for the percentage of cells in late stages of the cell cycle (S+G₂+M) by staining of cells with propidium iodide and analysis by flow cytometry as previously described by Thompson et al., *Nature,* 314:363-366 (1985).

As shown in Table 1, CD28 stimulation of CD3-stimutated T cells resulted in marked increases in cellular production of IL-2, TNF-α, IFN-γ and GM-CSF.

**TABLE 1**

| **Increased Cellular Production of T**_{**H**}**CD28 Lymphokines by CD28 Stimulation** | | | | | |
|---|---|---|---|---|---|
| STIMULUS | IL-2 (U/ml) | TNF-α/LT (U/ml) | IFN-γ (U/ml) | GM-CSF (U/ml) | S+G +M (%) |
| Medium | <2 | 0 | 0 | 0 | 4.6 |
| PMA | <2 | 0 | 0 | NT | 5.5 |
| anti-CD28 | <2 | 5 0 | | 0 | 6.5 |
| anti-CD28+PMA | 435 | 300 | 24 | 150 | 48.9 |
| anti-CD3ⁱ | 36 | 50 | 24 | 120 | 39.7 |
| anti-CD3ⁱ+anti-CD28 | 1200 | 400 | 74 | 1050 | 44.7 |
| lonomycin | <2 | 0 | 0 | NT | 6.6 |
| lonomycin+PMA | 200 | 5 | 37 | NT | 43.6 |
| lonomycin+PMA +anti-CD28 | 1640 | 320 | 128 | NT | 43.5 |
| ⁱ = immobilized | | | | | |
| NT = not tested | | | | | |

### B. Effects of Anti-CD28 Stimulation on T cell IL-4, IL-5 and IL-3 Secretion.

Previous studies of the effects of anti-CD28 mAb stimulation on T cell production of lymphokines of the T_{H}2 type were limited to the first few days of stimulation. In those studies IL-4 could not be detected after anti-CD28 stimulation (noted in Thompson et al., *PNAS* 86:1333 (1989)). On reexamination of this question, it was found that anti-CD28 can augment production of IL-4 and of IL-5, and thus, augments production of T_{H}2 lymphokines as well as the T_{H}1 type lymphokines previously described. As can be seen in Table 2, small amounts of both IL-4 and IL-5 can be detected after 24 h of stimulation with anti-CD3 plus anti-CD28. However, when cells are restimulated after 8 days in culture, large amounts of both IL-4 and IL-5 are secreted. As shown in Table 3, similar results were found when the CD28 subset of T cells were stimulated with the combination of phorbol myristate acetate (PMA) and anti-CD28 mAb. These results indicated that small amounts of IL-4 and IL-5 can be detected after initial stimulation of resting T cells with anti-CD28. However, with continued stimulation, differentiation occurs, and large amounts of IL-4 and, particularly, of IL-5 are produced, while lesser amounts of IL-2 and γ-IFN are also produced.

**TABLE 2**

| **Effects of anti-CD3 and anti-CD28 Treatment of IL-4 and IL-5 Production by T cells** | | | | | | | | |
|---|---|---|---|---|---|---|---|---|
| | INITIAL | | | RESTIMULATION | | | | |
| STIMULUS | IL-4 pg/ml | IL-5 pg/ml | IFN pg/ml | IL-2 U/ml | IL-4 pg/ml | IL-5 pg/ml | IFN pg/ml | IL-2 U/ml |
| Medium | <1 | <1 | <1 | <0.1 | <1 | <1 | <1 | <0.1 |
| anti-CD3 | <1 | <1 | 630 | 2.3 | 220 | 225 | 246 | 0.02 |
| anti-CD3 + anti-CD28 | 207 | 137 | 887 | 7.0 | 498 | 2545 | 379 | 0.2 |

The data in Table 2 above were obtained using the following protocol: CD28⁺ T cells were isolated by negative selection using monoclonal antibodies and magnetic immunobeads as described in Specific Example II. The cells were cultured at 1 X 10⁶/ml in RPMI medium containing 10% FCS (Medium), or in culture wells containing anti-CD3 monoclonal antibody G19-4 absorbed to the plastic, or plastic adsorbed anti-CD3 plus anti-CD28 mAb 9.3 added in solution at 0.5 µg/ml. Supernatants from the cell culture were analyzed for lymphokine concentration using commercially available EUSA kits and the values expressed as pg/ml for IL-4, IL-5 and γ-IFN, or as units per ml, for IL-2. Supernatants were analyzed after 24 h of culture (Initial) or alternatively, the cells were cultured for 8 days in the above forms of stimulation, the cells were recovered, washed, and then restimulated with their original treatment, and the supernatants analyzed after a further 24 h of stimulation (Restimulation). The values represent means of duplicate cultures.

**TABLE 3**

| **Effects of PMA and anti-CD28 Treatment on IL-4 and IL-5 Production by T cells** | | | | | | | | |
|---|---|---|---|---|---|---|---|---|
| | INITIAL | | | | RESTIMULATION | | | |
| STIMULUS | IL-4 pg/ml | IL-5 pg/ml | IFN pg/ml | IL-2 U/ml | IL-4 pg/ml | IL-5 pg/ml | IFN pg/ml | IL-2 U/ml |
| Medium | <1 | <1 | <1 | <0.1 | <1 | <1 | <1 | <0.1 |
| PMA | <1 | <1 | 152 | .07 | <1 | <1 | <1 | 0.6 |
| anti-CD28 + PMA | <1 | 187 | 558 | 8.1 | 285 | 392 | 335 | 10.3 |

The data in Table 3 above were obtained using the following protocol: CD28⁺ T cells were isolated by negative selection using monoclonal antibodies and magnetic immunobeads as described in Specific Example II. The cells were cultured at 1 X 10⁶/ml in RPMI medium containing 10% FSC (Medium), or in medium plus PMA 3 ng/ml, or in PMA plus anti-CD28 mAb 9.3 at 0.5 µg/ml. Supernatants from the cell culture were analyzed for lymphokine concentration using commercially available ELISA kits and the values expressed as pg/ml for IL-4, IL-5 and γ-IFN. Supernatants were analyzed after 24 h of culture (Initial) or, alternatively, the cells were cultured for 8 days in the above forms of stimulation, and then restimulated, and the supernatants analyzed after a further 24 h period of stimulation (Restimulation). The values represent means of duplicate cultures.

***Induction of IL-3 expression in T cells after anti-CD28 treatment.*** IL-3 is a multilineage hematopoietic growth factor that is primarily produced by T cells, and is generally considered to be produced by T_{H}1 cells. The experimental protocol and the findings described herein are described in detail in Guba, S.C. et al., *J Clin. Invest*. 84(6):1701-1706 (1989), incorporated herein by reference.

PBL were isolated as described previously. The CD28⁺ subset of T cells was isolated by negative selection as described by June, C.H. et al., *Mol. Cell. Biol*. 7:4472-4481 (1987). In some experiments, the CD28⁺ subset of T cells was isolated by incubating PBL with mAB 9.3, and then removing the CD28⁺ cells with goat anti-mouse coated magnetic beads (Advanced Magnetics Institute, Cambridge, MA). Northern (RNA) blot analysis was done as described by June, C.H. et al., *Mol. Cell. Biol*. 7:4472-4481 (1987). The IL-3 probe was a 1.0 kb Xho I cDNA fragment.

To determine if stimulation of the TCR/CD3 pathway of T cell activation induced IL-3 gene expression, CD28⁺ T cells were stimulated with maximal amounts of plastic immobilized anti-CD3 mAb in the presence or absence of 9.3 mAb 1 µg/ml for 1 to 36 h. As shown in Table 4, anti-CD28 resulted in a 3 to 5-fold augmentation of IL-3 mRNA expression over that induced by anti-CD3 alone. CD28 did not change the kinetics of IL-3 gene expression, which was at peak levels at 6 h after anti-CD3 or after anti-CD3 + anti-CD28 treatment. Further experiments showed that IL-3 gene expression was restricted to the CD28⁺ subset of T cells, as determined by Northern analysis (Table 4). The stability of IL-3 mRNA was also determined. T cells were treated for 3 h with anti-CD3 or anti-CD3 plus anti-CD28 mAb to induce IL-3 mRNA expression. At 3 h, actinomycin D was added to the culture to inhibit further RNA synthesis. Total cellular RNA was isolated, and the remaining IL-3 mRNA determined by Northern analysis. The half-life of IL-3 mRNA from anti-CD3 plus anti-CD28 treated cells was at least 8-fold longer than the IL-3 mRNA from anti-CD3 treated cells (Table 4). Thus, as would be expected from the previously described results of anti-CD28 on other lymphokines, it can be concluded that the effect of anti-CD28 on IL-3 gene induction can, in large part, be explained by the ability of anti-CD28 to stabilize the IL-3 messenger RNA.

**TABLE 4**

| CONDITION | IL-3 GENE EXPRESSION (arbitrary densitometry units) |
|---|---|
| EXPERIMENT #1 | |
| CD28⁺ T cells, 6 h, anti-CD3 | 1 |
| CD28⁺ T cells, 6 h, anti-CD3 + anti-CD28 | 3 - 5 |

| EXPERIMENT #2 | |
|---|---|
| CD28⁺ T cells, 8 h, PMA 3ng/ml + lonomycin 0.4µg/ml | >10 |
| CD28⁻ T cells, 8 h, PMA 3ng/ml + lonomycin 0.4 µg/ml | <1 |

| EXPERIMENT #3 | |
|---|---|
| CD28⁺ T cells, anti CD3, then actinomycin D 90 m | 1 |
| CD28⁺ T cells, anti-CD3 + anti-CD28, then actinomycin D 90 m | 8 |

### SPECIFIC EXAMPLE IV

### Comparison of CD28 Stimulation to Stimulation of Other T Cell Surface Molecules.

CD28⁺ T cells were cultured at approximately 1 x 10⁵ cells/well in RPMI media containing 5% heat-inactivated fetal calf serum (FCS), PHA 10 µg/ml, PMA 3 ng/ml, ionomycin at 100 ng/ml, anti-CD28 mAb 9.3 at 100 at ng/ml, or mAb 9.4 specific for CD45 at 1 µg/ml or mAb 9.6 specific for CD2 at 1 µg/ml, or immobilized mAb G19-4 specific for CD3 at 200 ng/well.

CD28⁺ T cells were cultured in quadruplicate samples in flat-bottomed 96-well microtiter plates in RPMI media containing 5% heat-inactivated fetal calf serum. Equal aliquots of cells were cultured for 18 h and then pulsed for 6 h with 1 µCi/well of ³H-uridine, or for 72 h and then pulsed for 6 h with 1 µCi/well of ³H-thymidine. The means and standard deviations (in cpm) were determined by liquid scintillation counting after cells were collected on glass fiber filters.

All cultures containing cells immobilized to plastic by anti-CD3 monoclonal antibodies were visually inspected to ensure complete cell harvesting. The failure of cells in these cultures to proliferate in response to PHA is the result of rigorous depletion of accessory cells, *in vivo* activated T cells, B cells, and CD11⁺ (CD28⁻) T cells by negative immunoabsorption as described in Specific Example II above. In each experiment, cells were stained with fluorescein-conjugated anti-CD2 mAb OKT11 and fluorescein-conjugated anti-CD28 mAb 9.3 and were shown to be over 99% and over 98% surface positive, respectively.

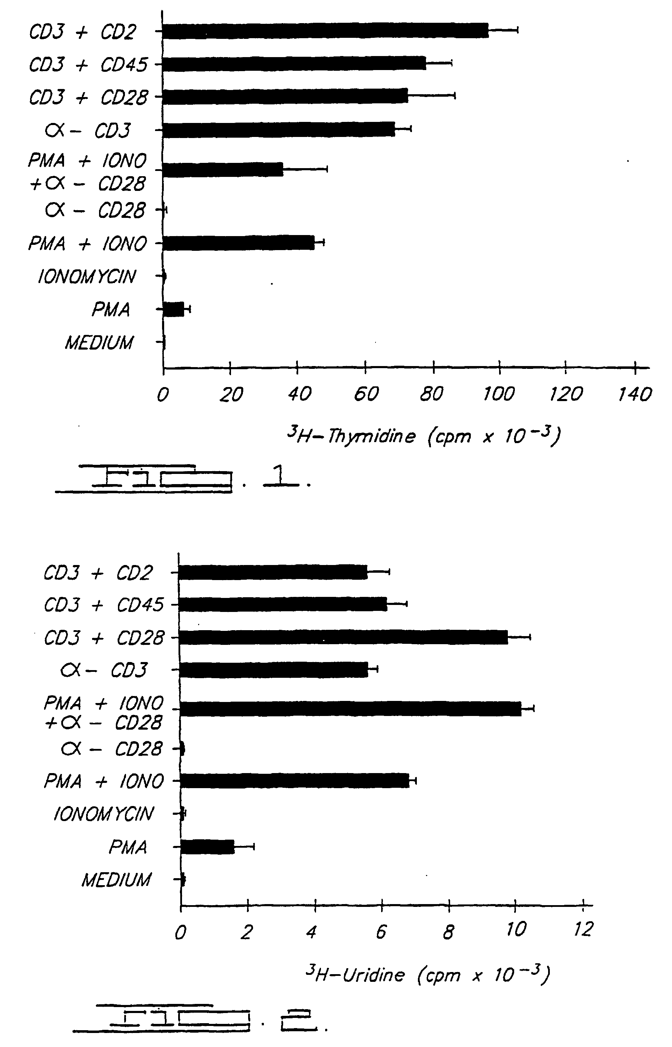

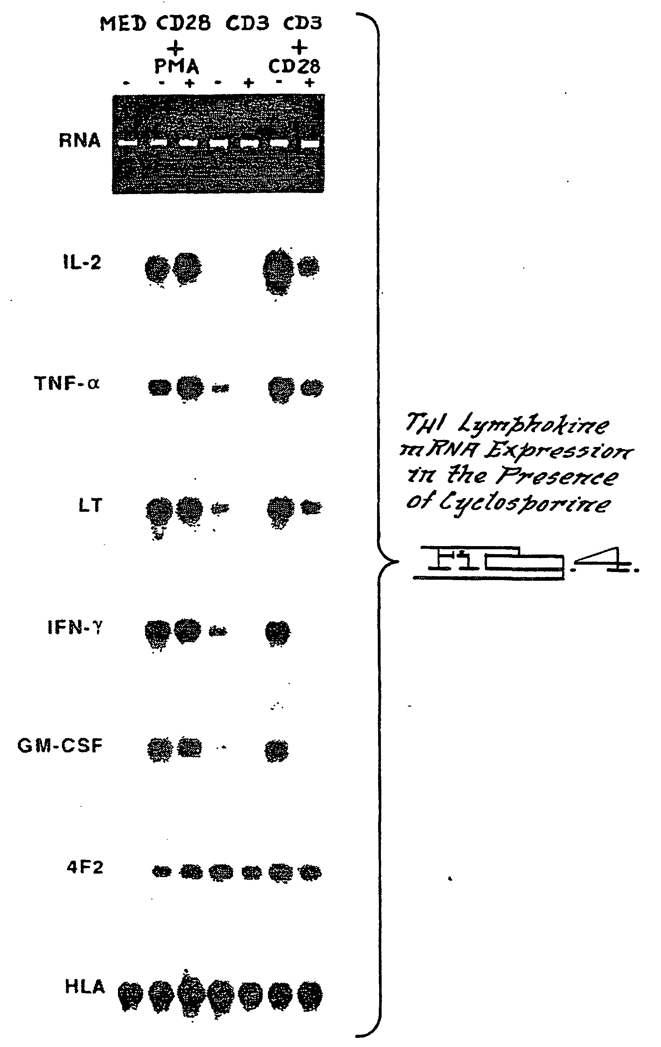

A representative experiment is illustrated in Figures 1 and 2. As shown in Figures 1 and 2, anti-CD28 by itself had no significant effect on uridine or thymidine incorporation, nor did it serve to augment proliferation induced either by immobilized anti-CD3 mAb G19-4 or chemically-induced T cell proliferation involving phorbol myristate acetate (PMA) and ionomycin (lono). However, as shown in Figure 2, anti-CD28 did significantly increase the uridine incorporation of both sets of cells. In contrast, other monoclonal antibodies including anti-CD2 mAb OKT11 and anti-CD45 mAb 9.4 had no significant effect on uridine incorporation of anti-CD3 stimulated cells. This was not due to lack of effect of these antibodies on the cells, since anti-CD2 monoclonal antibodies significantly augmented the proliferation of anti-CD3 stimulated cells. In separate experiments, the binding of isotype-matched mAbs to other T cell surface antigens (CD4, CD6, CD7 or CD8) failed to mimic the effects observed with anti-CD28.

These data serve to confirm that the stimulation of activated T cells by CD28 has a unique phenotype which appears to directly enhance the rate of incorporation of a radioactive marker into the steady state RNA of T cells without directly enhancing T cell proliferation.

### SPECIFIC EXAMPLE V

### Increased Cellular Production of Human T_{H}CD28 Lymphokines by CD28 Stimulation Ex Vivo.

Based on evidence from the *in vitro* systems it appeared that CD28 did not have a significant effect on cellular production of lymphokines unless they had undergone prior antigen activation or its equivalent. However, CD28 binding by the 9.3 mAb significantly enhanced the ability of anti-TCR/CD3 activated T cells to sustain production of human T_{H}1-type lymphokines. To test this effect in a physiologic setting, the activation of T lymphocytes in an *ex vivo* whole blood model was studied.

50-100 ml of venous blood was obtained by standard aseptic procedures from normal volunteers after obtaining informed consent. The blood was heparinized with 25 U/ml of preservative-free heparin (Spectrum, Gardenia, CA) to prevent clotting. Individual 10 ml aliquots were then placed on a rocking platform in a 15 ml polypropylene tube to maintain flow and aeration of the sample.

To assay for the effectiveness of CD28 stimulation on the induction of lymphokine gene expression, the production of TNF-α molecule was chosen as a model because of the extremely short half-life (approximately 15 minutes) of the protein in whole blood. 10 ml of whole blood isolated as described above was incubated with soluble anti-CD3 mAb G19-4 at a concentration of 1 µg/ml or anti-CD28 mAb 9.3 at a concentration of 1 µg/ml or a combination of the two antibodies. The plasma was assayed for TNF-α as described in Specific Example III at one and four h. An example of one such experiment is shown in Table 5, which illustrates the significant increase in sustained production of TNF-α by maximal stimulation of CD3 and costimulation of CD28.

**TABLE 5**

| STIMULUS | TNF-α (pg/ml) | | |
|---|---|---|---|
| | 0 h | 1 h | 4 h |
| anti-CD3 | 4.5^{a} | 65.0 | 2.1 |
| anti-CD28 | 4.5^{a} | 1.6 | 3.3 |
| anti-CD3+anti-CD28 | 4.5^{a} | 35.0 | 75.0 |

| | | | |
|---|---|---|---|
| ^{a} value determined prior to addition of monoclonal antibody to aliquots of the venous sample | | | |

### SPECIFIC EXAMPLE VI

### Resistance of CD28-Induced T Cell Proliferation to Cyclosporine.

The protocol used and results described herein are described in detail in June, C.H. et al., *Mol. Cell. Biol*., 7: 4472-4481 (1987), herein incorporated by reference.

T cells, enriched by nylon wool filtration as described by Julius, et al., *Euro. J. Immunol*., 3:645-649 (1973), were cultured at approximately 5 x 10⁴/well in the presence of stimulators in the following combinations: anti-CD28 mAb 9.3 (100ng/ml) and PMA 1 (ng/ml); or immobilized anti-CD3 mAb G19-4 (200ng/well); or PMA (100ng/ml). The above combinations also included fourfold titrations (from 25ng/ml to 1.6µg/ml) of cyclosporine (CSP) (Sandoz, Hanover, NJ) dissolved in ethanol-Tween 80 as described by Wiesinger, et al., *Immunobiol*., 156:454-463 (1979).

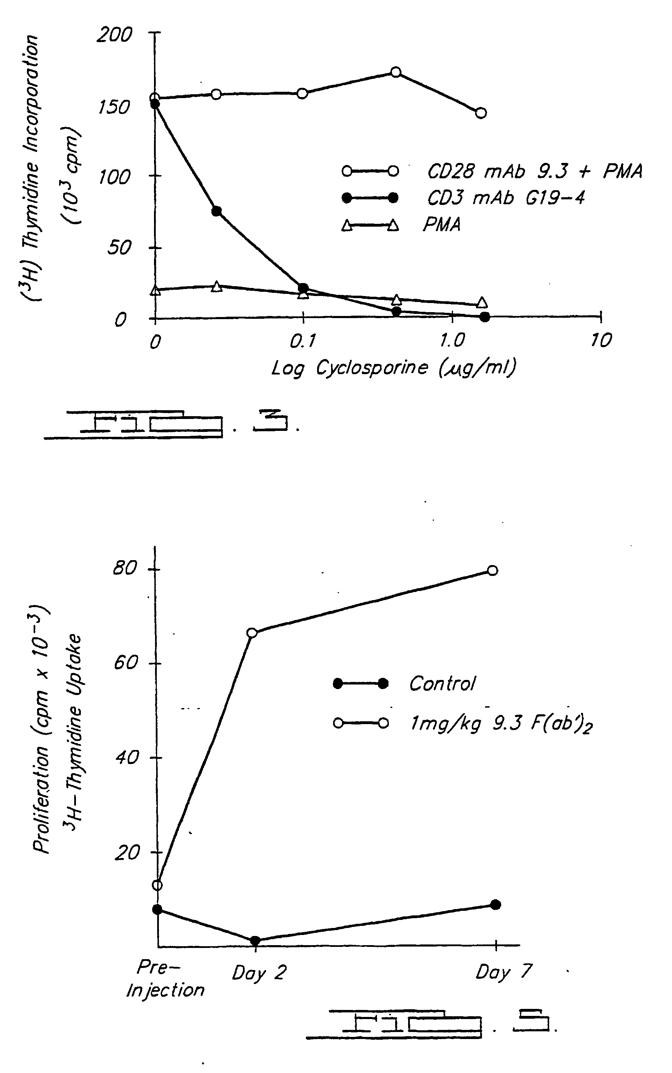

³H-thymidine incorporation was measured on day 3 of culture and the results representative of eight independent experiments are depicted in Figure 3. The arithmetic mean (91,850 ± 1300 (mean ± SD)) CD28-induced T cell proliferation exhibits nearly complete cyclosporine resistance when accompanied by the administration of PMA. Table 6 below illustrates the effects of cyclosporine on CD3-induced proliferation of CD28⁺ T cells cultured at approximately 5 x 10⁴ cells/well in flat-bottomed 96-well microtiter plates (CoStar, Cambridge, MA) under the following conditions: immobilized mAb G19-4; or immobilized mAb G19-4 and mAb 9.30 100ng/ml; or immobilized mAb G19-4 and PMA 1 ng/ml; or mAb 9.3 100ng/ml and PMA 1 ng/ml. Cyclosporine was prepared as above and included in the cultures at 0, 0.2, 0.4, 0.8, 1.2 µg/ml.

³H-thymidine incorporation was determined on day 3 of culture as above. The percent inhibition of proliferation was calculated between CD28⁺ T cells cultured in medium only or in cyclosporine at 1.2 µg/ml. CD28⁺ T cells cultured in the absence of cyclosporine were given cyclosporine diluent. ³H-thymidine incorporation of cells cultured in medium, or PMA, or monoclonal antibody 9.3 only were less than 150 cpm. As shown in Table 6, costimulation of CD3 and CD28 resulted in a marked increase in the resistance of T cell proliferation to cyclosporine and the stimulation of CD28 in the presence of PMA resulted in a complete absence of cyclosporine suppression of T cell proliferation. As shown in Table 7, stimulation of CD28 together with immobilized anti-CD3 also resulted in resistance to suppression of T cell proliferation by the immunosuppressant dexamethasone.

**TABLE 7**

| Effects of CD28 Stimulation on Dexamethasone Resistance on T cell Proliferation | | | |
|---|---|---|---|
| ³HTdR + Dexamethasone (nM) | | | |
| STIMULUS | 0 | 25 | %INHIBIT |
| CD3 mAb G194 | 14,700 | 770 | 97 |
| CD3 mAb + IL-2 | 21,700 | 1,900 | 93 |
| CD28 mAb + PMA | 181,600 | 197,700 | <0 |
| PMA | 5,000 | 1,400 | 72 |

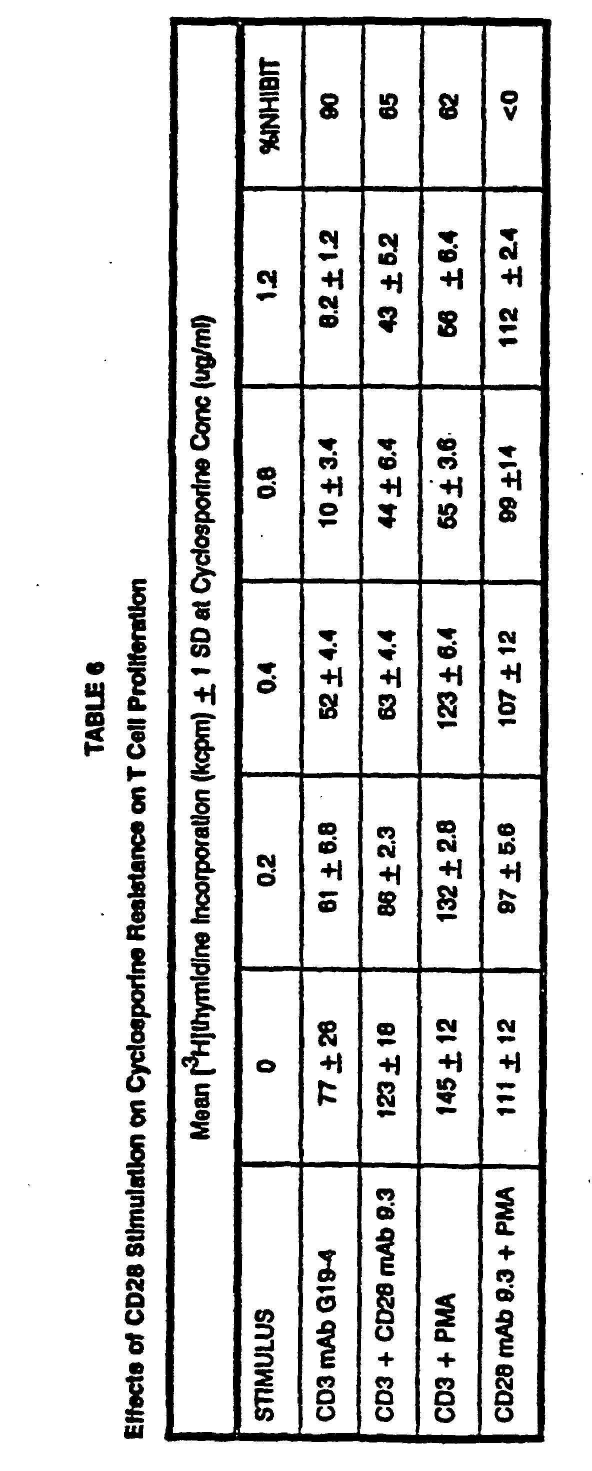

### SPECIFIC EXAMPLE VII

### Human T_{H}CD28 Lymphokine Secretion in the Presence of Cyclosporine.

As described in Specific Example III, CD28⁺ T cells were cultured in the presence of various stimulators. Culture supernatants were harvested at 24 h and serial dilutions assayed for IL-2, TNF-α/LT, IFN-γ, and GM-CSF as previously described. Separate aliquots of cells were recovered 48 h after stimulation and assayed for the percentage of cells in late stages of the cell cycle (S+G₂+M).

When cyclosporine at 0.6µg/ml was included in the test protocol, as shown in Table 8 (which also incorporates the data of Specific Example III for comparison), CD28⁺ T cells were found to secrete the T_{H}CD28 lymphokines in the presence of cyclosporine in cultures stimulated with mAb 9.3 and PMA; or immobilized mAb G19-4 and mAb 9.3; or PMA and ionomycin and mAb 9.3. T_{H}CD28 lymphokine production induced by immobilized mAb G19-4; or by PMA with ionomycin was, however, completely suppressed in the presence of cyclosporine.

**TABLE 8**

| STIMULUS | IL-2 (U/ml) | TNF-α/LT (U/ml) | IFN-γ (U/ml) | GM-CSF (U/ml) | S+G₂+M (%) |
|---|---|---|---|---|---|
| Medium | <2 | 0 | 0 | 0 | 4.6 |
| PMA | <2 | 0 | 0 | NT | 5.5 |
| anti-CD28 | <2 | 5 | 0 | 0 | 6.5 |
| anti-CD28+PMA | 435 | 300 | 24 | 150 | 48.9 |
| anti-CD28+ PMA+CSP | 192 | 200 | 12 | NT | 49.3 |
| anti-CD3ⁱ | 36 | 50 | 24 | 120 | 39.7 |
| anti-CD3ⁱ+CSP | <2 | 0 | 0 | NT | 14.5 |
| anti-CD3ⁱ+anti-CD28 | 1200 | 40 | 74 | 1050 | 44.7 |
| anti-CD3ⁱ+anti-CD28+CSP | 154 | 200 | 9 | NT | 48.6 |
| lonomycin | <2 | 0 | 0 | NT | 6.6 |
| lonomycin+PMA | 200 | 5 | 37 | NT | 43.6 |
| lonomycin+ PMA+CSP | <2 | 0 | 0 | NT | 8.1 |
| lonomycin+ PMA+anti-CD28 | 1640 | 320 | 128 | NT | 43.5 |
| lonomycin+PMA+ anti-CD28+CSP | 232 | 120 | 15 | NT | 47.6 |
| ⁱ = immobilized | | | | | |
| NT = not tested | | | | | |

### SPECIFIC EXAMPLE VIII

### Human T_{H}CD28 Lymphokine mRNA Expression in the Presence of Cyclosporine.

In order to further examine whether CD28 stimulation led to cyclosporine-resistant T_{H}CD28 lymphokine gene expression as well as secretion, the ability of cyclosporine to suppress induction of IL-2, TNF-α, LT, IFN-γ, and GM-CSF following stimulation by various stimulators was tested. Specifically, CD28⁺ T cells were cultured at 2 x 10⁶/ml in complete RPM1 medium (GIBCO, Grand Island, NY) with 5% FCS (MED). Individual aliquots of CD28⁺ T cells were incubated for 6 h in the presence or absence of 1.0 µg/ml cyclosporine with PMA 3ng/ml and anti-CD28 mAb 9.3 (1 mg/ml); or with immobilized anti-CD3 mAb G19-4 (1 µg/well); or with immobilized mAb G19-4 (1µg/well) and mAb 9.3 (1 ng/ml). CD28⁺ T cells were harvested, total cellular RNA isolated and equalized for ribosomal RNA as previously described by Thompson, et al., *Nature,* 314:363-366 (1985).

Northern blots were prepared and hybridized sequentially with ³²P-labeled, nick-translated gene specific probes as described by June, C.H. et al., *Mol. Cell. Biol*., 7:4.472-4481 (1987). The IL-2 probe was a 1.0 kb Pst I cDNA fragment as described by June, C.H. et al., *Mol. Cell. Biol*., 7:4472-4481 (1987); the IFN-γ probe was a 1.0 kb Pst I cDNA fragment as described by Young, et al., *J. Immunol*., 136:4700-4703 (1986). The GM-CSF probe was a 700 base pair EcoR I-Hind III cDNA fragment as described by Wong, et al., Science, 228:810-815 (1985); the 4F2 probe was a 1.85 kb EcoR I cDNA fragment as described by Lindsten, et al., *Mol. Cell. Biol*., 8:3820-3826 (1988); the IL4 probe was a 0.9 kb Xho I cDNA fragment as described by Yokota, et al., *PNAS (USA),* 83:5894-5898 (1986); and the human leukocyte antigen (HLA) probe was a 1.4 kb Pst I fragment from the HLA-B7 gene as described by Lindsten, et al., *Mol*. *Cell. Biol*., 8:3820-3826 (1988). TNF-α and LT specific probes were synthesized as gene-specific 30 nucleotide oligomers as described by Steffen, et al., *J. Immunol*., 140:2621-2624 (1988) and Wang, et al., *Science,* 228:149-154 (1985). Following hybridization, blots were washed and exposed to autoradiography at -70°C. Quantitation of band densities was performed by densitometry as described in Lindsten, et al., *Mol. Cell. Biol*., 8:3820-3826 (1988).

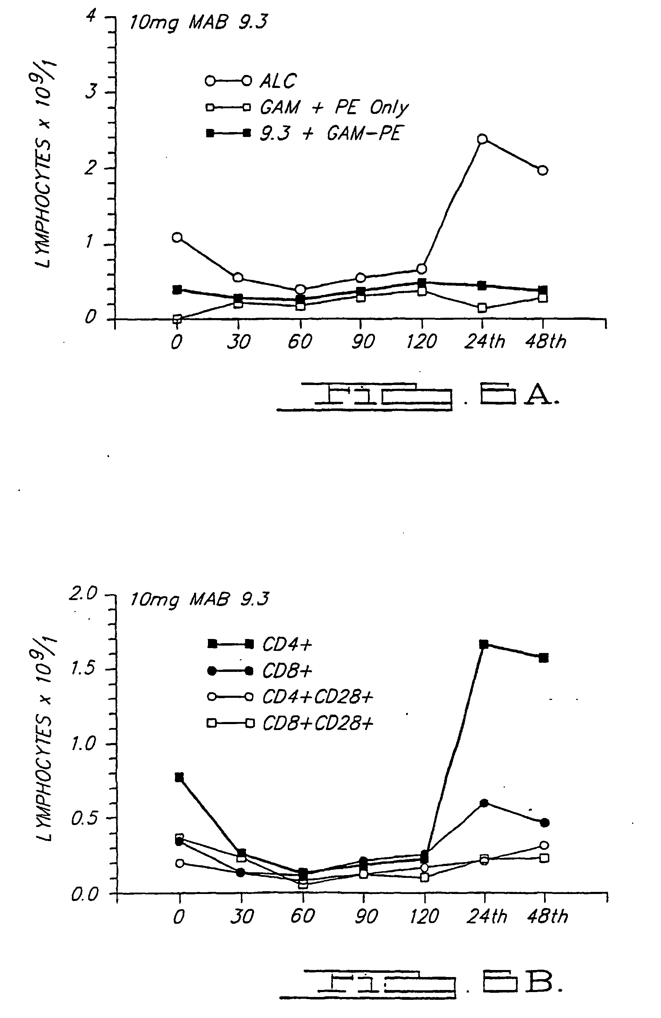

As illustrated by the Northern blot of Figure 4, stimulation by mAb 9.3 with PMA and by mAb 9.3 with mAb G19-4 led to human T_{H}CD28 lymphokine gene expression that exhibited resistance to cyclosporine. In contrast, stimulation by TCR/CD3 mAb G19-4 alone was completely suppressed in the presence of cyclosporine.

### SPECIFIC EXAMPLE IX

### In Vivo Activation of T Cells by CD28 Stimulation.

### A. Monoclonal Antibody 9.3 F(ab')₂.

F(ab')₂ fragments of mAb 9.3 were prepared as described by Ledbetter, J. A. et al., *J. Immunol*., 135:2331-2336 (1985). Purified and endotoxin-free F(ab')₂ fragments were injected intravenously at 1 mg/kg of body weight over a 30 minute period into a healthy macaque (*M. nemestrina*) monkey. On days 2 and 7 after injection, 5 ml of blood was drawn and tested.

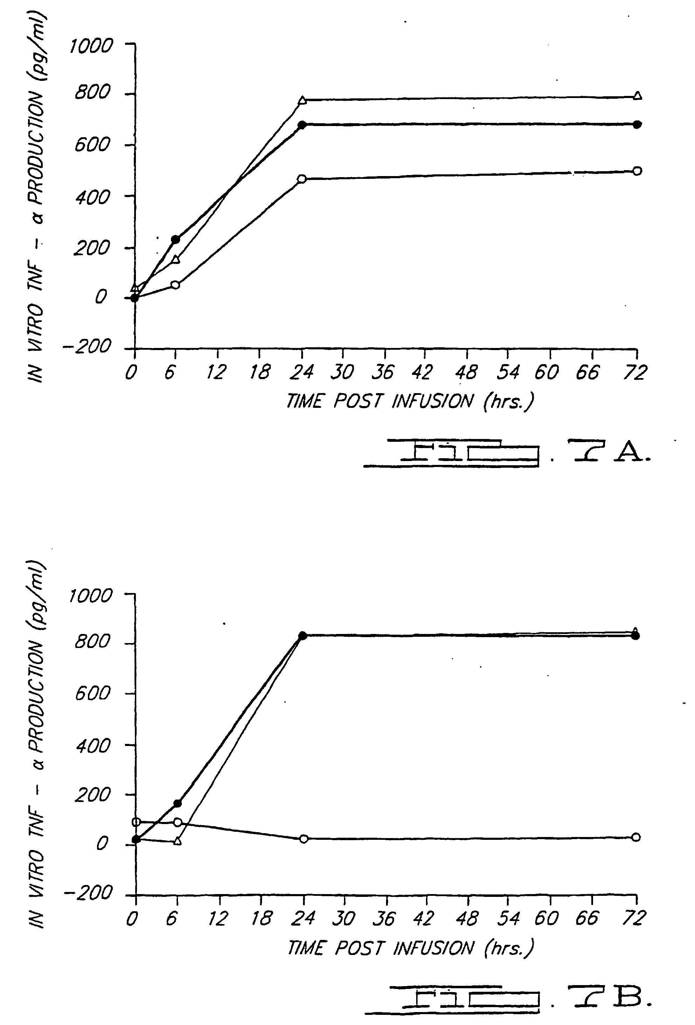

Peripheral blood lymphocytes from the monkey's blood were isolated by density gradient centrifugation as described in Specific Example II. Proliferation of peripheral blood mononuclear cells in response to PMA (1ng/ml) was tested in the treated monkey and a control animal (no F(ab')₂ fragment treatment) in triplicate as described in Specific Example IV. Proliferation was measured by the uptake of ³H-thymidine during the last 6 h of a three-day experiment and the results shown in Figure 5. Means of triplicate culture are shown, and standard errors of the mean were less than 20% at each point. As shown in Figure 5, *in vivo* stimulation of CD28 by the F(ab')₂ mAb 9.3 fragment increased T cell proliferation for at least 7 days.

### B. Monoclonal Antibody 9.3

Two doses of mAb 9.3, 10mg and 0.1mg, were administered intravenously to primates *Macaca mulatta*. Three animals were evaluated as described below at each dose.

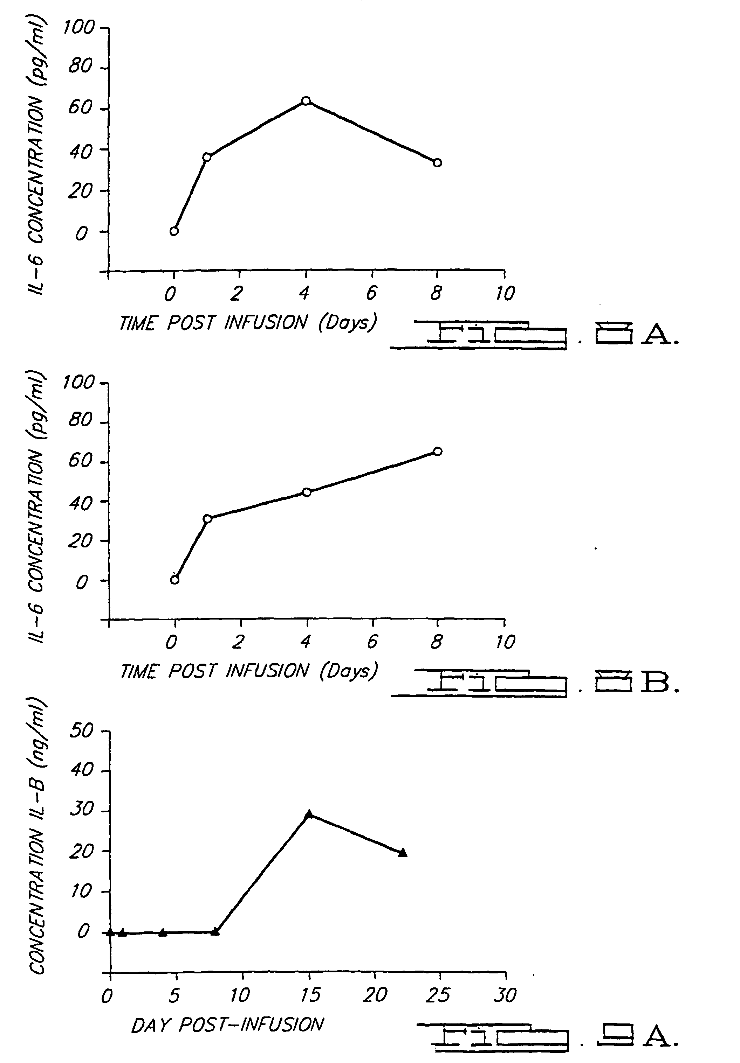

***Cell population changes*.** At the higher dose, immediate effects were monitored over the first 120 m. In Figures 6A and 6B, a representative result is depicted showing the change in the lymphocyte counts over time. The ALC and distribution of CD28⁺ cells are depicted in Figure 6A, while Figure 6B illustrates the distribution of CD4⁺ and CD8⁺ cells. The absolute lymphocyte count (ALC) decreased over the first 60 m and then increased at 24 h (see Figure 6A). In this case, the number of CD28 positive lymphocytes remained essentially the same, but was coated with antibody after the first 30 m (as determined by adding goat anti-mouse phycoerythrin). In this same animal, the CD4 and CD8 positive populations were followed and the increase at 24 h was the result of CD8⁺CD28⁻ cells (see Figure 6B). Animals re-evaluated after 8 days had between 35 to 60% of the CD28⁺ cells coated with antibody. There was no significant change in the circulating lymphocyte counts in primates treated with 0.1mg.

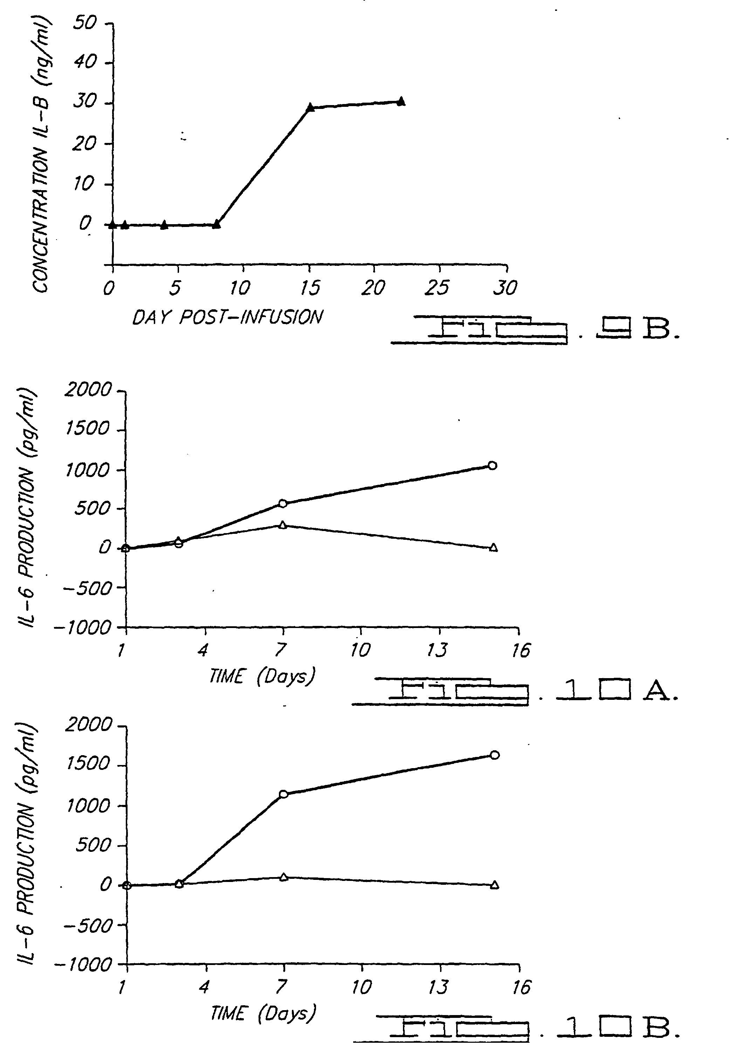

***Cytokine released after in vitro stimulation*.** PBLs isolated at specific time points from primates previously immunized with tetanus toxoid and treated with 10mg mAb 9.3 were cultured *in vitro* to determine the effect of antigenic stimulation on cytokine production. Figure 7A represents the *in vitro* production of TNF while Figure 7B represents the *in vitro* production of IL-6. PBLs stimulated with Concanavalin-a (Con-a) are depicted by Δ. PBLs stimulated with tetanus toxoid (TT) are depicted by •. Unstimulated PBLs are depicted by ○. As shown in Figures 7A and 7B, cultures of baseline cells did not respond to either Con-a or TT stimulation. However, as shown in Figure 7A, PBLs isolated from animals 6 h after infusion of antibody showed an increase in TNF production. As depicted in Figure 7B, after 24 h, unstimulated cultures produced TNF but not IL-6. TT stimulation of PBLs produced a similar quantity of both TNF and IL-6 as mitogen stimulated cultures. This response was consistent through 72 h.

Monoclonal antibody 9.3 was administered to the primates either as a single day bolus of 10mg (n=3) or as multiple daily injections of 10 mg/d for 5 consecutive days (n=3) and the animals were followed simultaneously. The changes in the peripheral blood cell populations were not dramatic. ALC as previously observed decreased with the first injection but recovered to above baseline if no further injections were administered. However, for animals treated with multiple injections, ALC remained -25% below normal during the period of mAb administration. ALC in multiple-treated animals did not recover to above normal levels over the 21-day study period. Absolute neutrophil count decreased by -30% in the 5 day treated group.

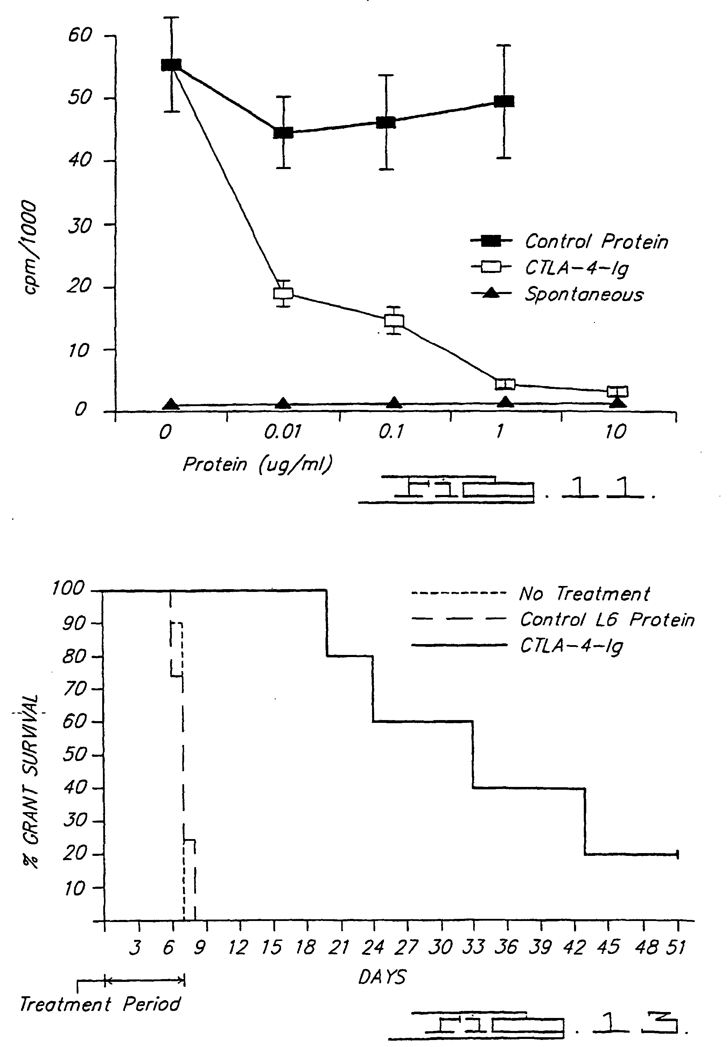

***Cytokine levels in serum*.** Serum was analyzed for IL-6, TNF, and IL-1 β. The detection of TNF from the serum preparations was not successful and therefore no results are available at this time. Figures 8A and 8B demonstrate the serum concentration of IL-6 after infusion of mAb 9.3. As shown in Figures 8A and 8B, increased IL-6 levels 24 h after mAb infusion were detected. In animals injected one time, the IL-6 levels increased to a peak on day 4, but a decrease was observed when remeasured on day 8 (see Figure 8A). In comparison, 5 day treated animals (multiple doses) demonstrated continual increase(s) in IL-6 through day 8 (see Figure 8B).

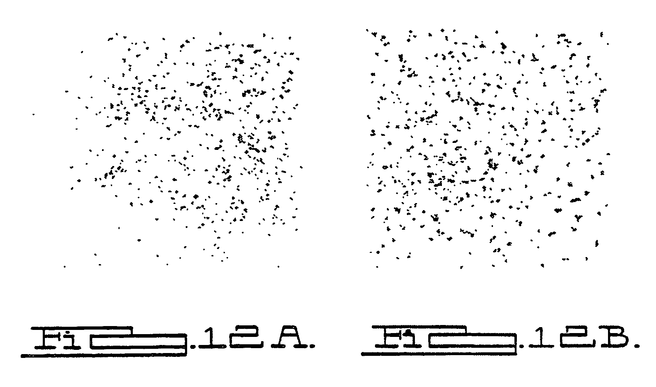

Figures 9A and 98 demonstrate the serum concentration of IL-1 after infusion of mAb 9.3. As shown in Figures 9A and 9B, measurements of IL-1p in the serum, did not detect any IL-1 until after day 8 in both single injected animals (see Figure 9A) and multiple injected animals (see Figure 9A). The multiple injected animals however had increasing levels at day 21 versus decreased levels for single injected animals.

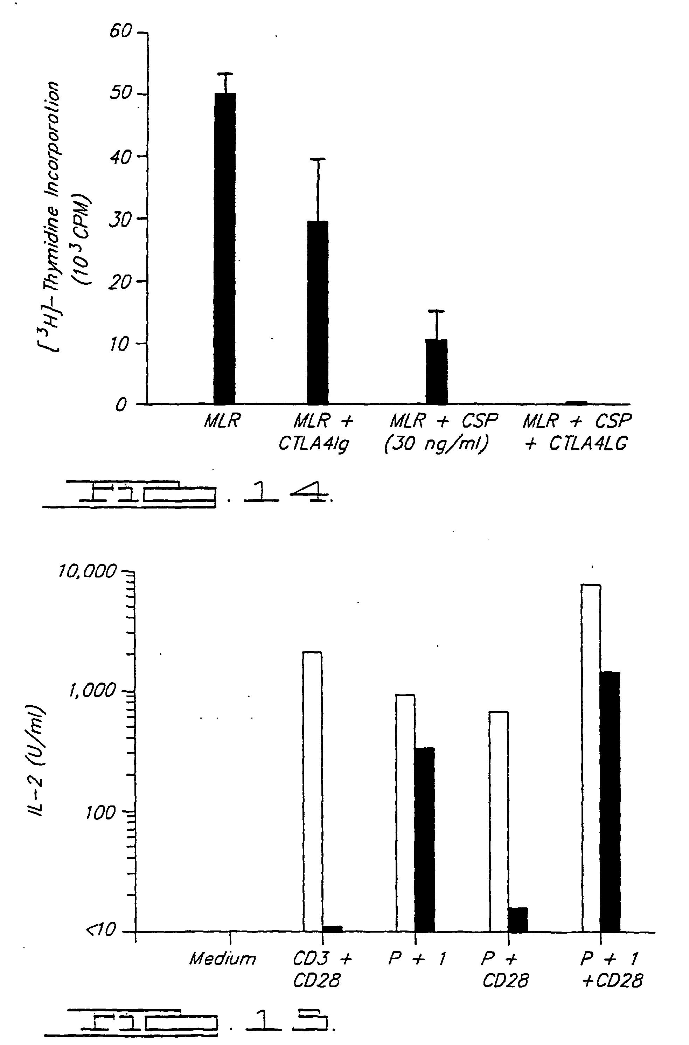

***Cytokine release after in vitro stimulation*.** IL-6 production was not detected in the PBLs of animals on day 3 after *in vitro* stimulation with TT. (Figure 10). This contrasted the previous *in vitro* results. However, an increased production of IL-6 was detected on day 7 and the more significant increase was observed from PBLs isolated from the 5 day treated primates. This increase in production was further observed in culture of PBLs isolated on day 14.

***IL-6 Production and Proliferation of PBLs*.** Figures 10A and 10B illustrate IL-6 production of *in vitro* stimulated PBLs isolated from monkeys. Days 1, 3 and 14 are depicted in Figures 10A and 10B with Δ representing the control and ○ representing the stimulated PBL response. Figure 10A illustrates the response of a single injected animal and Figure 10B illustrates the response of a multiple injected animal. (Note that the quantity of cells harvested from PB limited the number of assays performed, resulting in no day zero points and no day zero data.) PBLs were isolated from the different treated groups and evaluated for their proliferative response to Con-a, TT and no stimulus. Historically the TT response was -5,000cpm and the Con-a response was -35,000cpm. Thus, the results indicate that the PBL proliferative response to Con-a was reduced and gradually recovered over time. No proliferative response was observed when stimulated with TT. This contrasts the lymphokine production observed in *in vitro* cultures.

### SPECIFIC EXAMPLE X

### Immunoregulation with CTLA4Ig.

### A. In Vitro.

The effects of CTLA4Ig on the primary immune response to alloantigen was initially examined in a one-way mixed lymphocyte culture (MLC) between Lewis rats (RT1^{l}, responder) and Brown-Norway rats (RT1ⁿ, stimulator). Lymphocytes were isolated from paratracheal and cervical lymph nodes. Cultures were performed in quadruplicate in 96-well round bottomed plates as described in Turka, L.A. et al., *Transplant*., 47:388-390 (1989). Cultures were harvested after 4 days and 1mCi/well of ³H-thymidine was added for the last 6 h of culture. In this assay, Brown-Norway stimulator cells were irradiated at 30 Gy to prevent their proliferation and then added to cultures of Lewis responder lymphocytes. A proliferative response will normally occur in approximately 1-5% of cells as a result of activation through their cell-surface TCR in response to allogeneic MHC as discussed in Marrack, P. et al., *Immunol. Today*, 9:308-315 (1988). Graded concentrations of CTLA4Ig or an isotype-matched control monoclonal antibody L6 described in Fell, H.P. et al., *J. Bio. Chem.* (in press), was added.

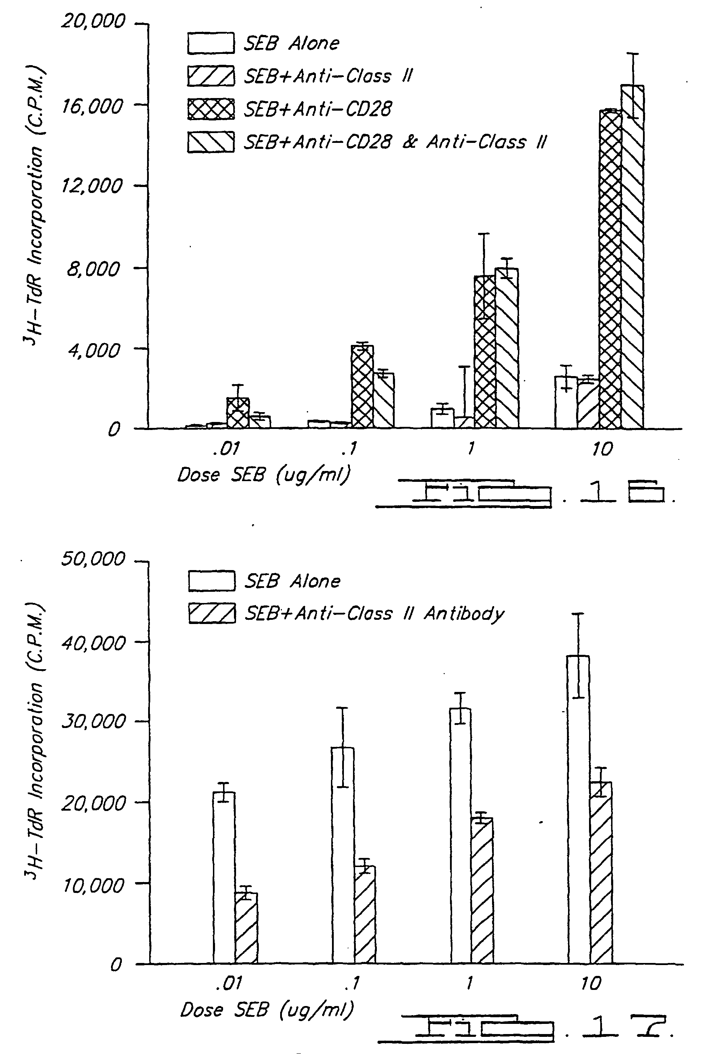

Figure 11 represents the effect of CTLA4Ig on a one-way mixed lymphocyte culture. As shown in Figure 11, CTLA4Ig was able to block proliferation in a dose dependent fashion with virtually complete inhibition observed at a concentration of 1 mg/ml. (Results are expressed as counts per minute of ³H-thymidine incorporation ± standard deviation). Spontaneous proliferation is the incorporation of thymidine by Lewis cells in the absence of Brown-Norway stimulators which is depicted as a closed square in Figure 11. Consistent with these results, alloreactive T cell responses can also be inhibited by non-stimulatory F(ab') fragments of an anti-CD28 monoclonal antibody as shown in Azuma, M. et al., *J. Exp. Med*., 175:353-360 (1992). Together, these data suggest that in order to mount a proliferative response *in vitro*, alloreactive T cells must be stimulated not only through MHC engagement of the TCR but also require costimulation by B7 engagement of the CD28 receptor.

### B. In Vivo: Cardiac Allografts.

CTLA4Ig was next used in a rat model of organ transplantation to ascertain its ability to block alloantigen responses *in vivo*. Recipient animals received a heterotopic cardiac allograft which was anastamosed to vessels in the neck as described in Boiling, S.F. et al., *Transplant*, 53:283-286 (1992). Grafts were monitored for mechanical function by palpation and for electrophysiologic function by electrocardiogram. Graft rejection was said to occur on the last day of palpable contractile function. As an initial test, animals were treated with daily injections of CTLA4Ig or an isotype-matched control monoclonal antibody L6 for 7 days. CTLA4Ig was administered at doses of 0.015 mg/day (5 animals), 0.05 mg/day (5 animals), and 0.5 mg/day (8 animals). L6 was given at 0.5 mg/day. Untreated Lewis rats rejected the heterotopic Brown-Norway allografts in 6.8 ± 0.3 days (n=10). The allografts in CTLA4Ig-treated animals remained functional following completion of drug administration, whereas untreated animals, or animals treated with the L6 control antibody, uniformly rejected their grafts by day 8 (p<0.0001) as shown in Table 9. (p values were calculated by Chi-square analysis).

**TABLE 9**

| GRAFT SURVIVAL DAY 8 | | SIGNIFICANCE |
|---|---|---|
| Untreated | 0/10 | |
| CTLA4Ig | 18/18 | p<0.0001 |
| Control Protein | 0/5 | p<0.0001 |

CTLA4Ig-treated rats manifested no observable acute or chronic side effects from administration of the protein. No gross anatomic abnormalities were observed in CTLA4Ig-treated animals at autopsy.

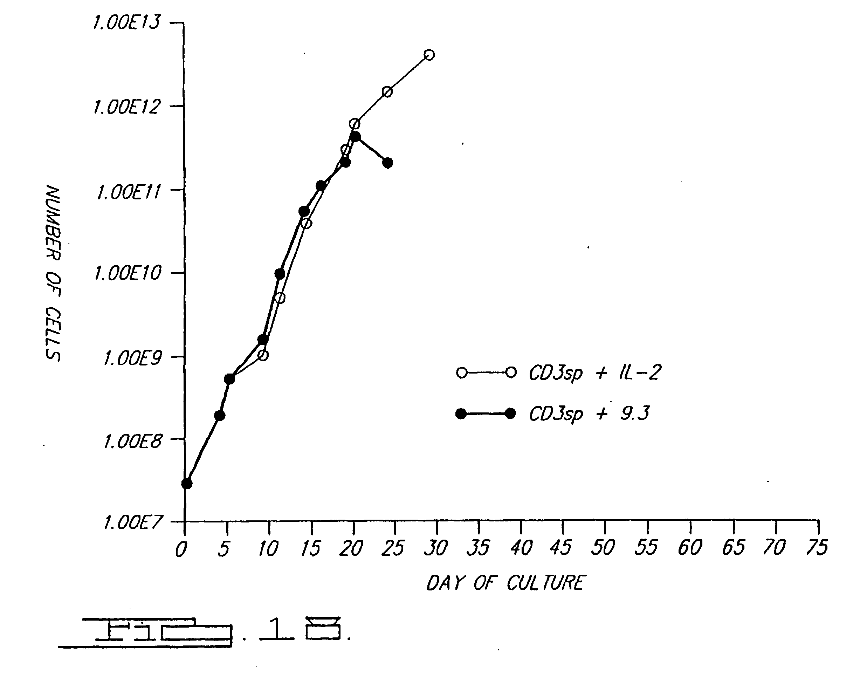

An untreated animal and a CTLA4Ig-treated animal were sacrificed for histological examination. Cardiac allografts were removed from an untreated animal (shown in Figure 12A) and a CTLA4Ig-treated animal (0.5 mg/day) (shown in Figure 12B) four days after transplantation. Allografts were fixed in formalin, and tissue sections were stained with hematoxylin-eosin. (Original photography at 200X magnification.) The donor heart removed from the untreated animal showed histological findings of severe acute cellular rejection, including a prominent interstitial mononuclear cell infiltrate with edema formation, myocyte destruction, and infiltration of arteriolar walls. In contrast, the transplanted heart from the CTLA4Ig-treated animal revealed only a mild lymphoid infiltrate. Frank myocyte necrosis and evidence of arteriolar involvement were absent. The native heart from each animal showed no histological abnormalities.

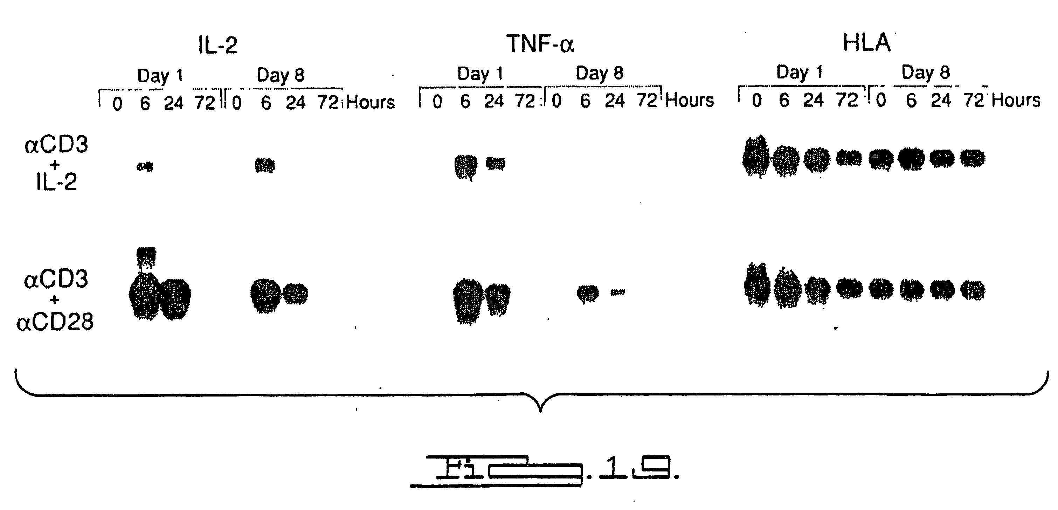

To determine whether CTLA4Ig therapy established a state of graft tolerance that persisted following drug treatment, animals treated for 7 days with daily injections of CTLA4Ig were observed without additional therapy until cessation of graft function. Animals received either no treatment, CTLA4Ig (0.5 mg/day x 7 days), or an isotype-matched control monoclonal antibody, L6 (0.5 mg/day x 7 days). In all cases treatment was initiated at the time of transplantation. Figure 13 is a graph showing allograft survival in the treated and control rats. Graft survival was 18 - 40 days in animals treated with 0.05 mg/day of CTLA4Ig. Graft survival was assessed daily. This failure to induce permanent engraftment did not appear to be due to inadequate dosing of CTLA4Ig, as animals treated with a ten-fold higher dose, 0.5 mg/day, showed a similar graft survival curve as depicted in Figure 13, with one animal maintaining long-term graft function (>50 days). In Figure 13, graft survival is displayed as the last day of graft function. Animals treated with a dose of 0.015 mg/day x 7 days (n=5) had a mean survival of 12.6 ± 2.1 days (n=5). Furthermore, serum CTLA4Ig trough levels in this group as measured in a quantitative ELISA assay were in excess of 10 µg/ml, a concentration which is maximally suppressive *in vitro* (see Figure 11). Histological examination of the allografts from CTLA4Ig-treated animals whose grafts ceased functioning after 18-43 days displayed typical signs of acute cellular rejection, of the same degree of severity as seen in control animals that had rejected their hearts after 7 days. The animal with continued graft function was sacrificed on day 57, and the allograft from this animal failed to reveal any histological abnormalities.

At the time of sacrifice, lymphocytes from the day 57 "tolerant" animal, and from a CTLA4Ig-treated animal that rejected the heart at day 33, were tested for their functional responses. These responses were compared with those of lymphocytes from a control (non-transplanted) Lewis rat, and results were normalized as a percentage of the control response. In comparison to control animals, lymphocytes from both the "tolerant" and rejecting animal had equivalent proliferative responses to Con-a, (tolerant, 62.5%; rejecting, 51.1 %; p=0.63, two-tailed T test) and to cells from a third party ACI rat (RT1 ^{avl}) (tolerant, 160%; rejecting, 213%; p=0.58). However a significant disparity was seen in the response to Brown-Norway cells (tolerant, 34.2%; rejecting, 238%; p<0.005), suggesting that T cells from animals with functioning grafts were specifically hyporesponsive to donor MHC antigens. The thymus and spleen from the day 57 "tolerant" animal were similar in size and cell number to the non-transplanted control rat, and flow cytometric analyses of thymus, lymph nodes and spleen revealed similar percentages of both CD4⁺ and CD8⁺ T cells in each animal. Splenocytes adoptively transferred from the day 57 "tolerant" animal into a native Lewis recipient failed to affect the rate at which that animal rejected a Brown-Norway cardiac allograft. Thus, tolerance did not appear to be maintained by suppressor cells in this animal.

The fact that 4 of 5 allograft recipients treated with high dose CTLA4Ig (0.5 mg/day x 7 days) rejected their grafts within the study period indicated that blockade of the costimulatory molecule B7 did not consistently induce permanent graft tolerance. One possible explanation was that CTLA4Ig induced a temporary state of non-responsiveness, and that upon recovery, recipient T cells could effect graft rejection. Alternatively, CTLA4Ig treatment may have resulted in a state of permanent non-responsiveness in circulating T cells by allowing target antigen recognition without B7-dependent costimulation. Newly matured T cells emerging from the thymus after cessation of CTLA₄Ig treatment could not be tolerized by this mechanism, and could mediate graft rejection as a result of B7-costimulated T cell alloreactivity. To differentiate between these two possibilities, rats were thymectomized 3 days prior to cardiac transplantation, and treated with daily injection of CTLA4Ig (0.5 mg/day x 7 days) following transplantation. These animals rejected their grafts between days 28 and 33, indicating that allograft recipients were not dependent upon the influx of new T cells to initiate an alloimmune response. Thus, it appears that T cells present during the time of CTLA4Ig treatment can eventually induce graft rejection. This may represent T cell recovery from a temporary state of non-responsiveness, or may reflect the kinetics of T cell trafficking during the CTLA4Ig treatment period.

### C. Synergistic effects with cyclosporine.

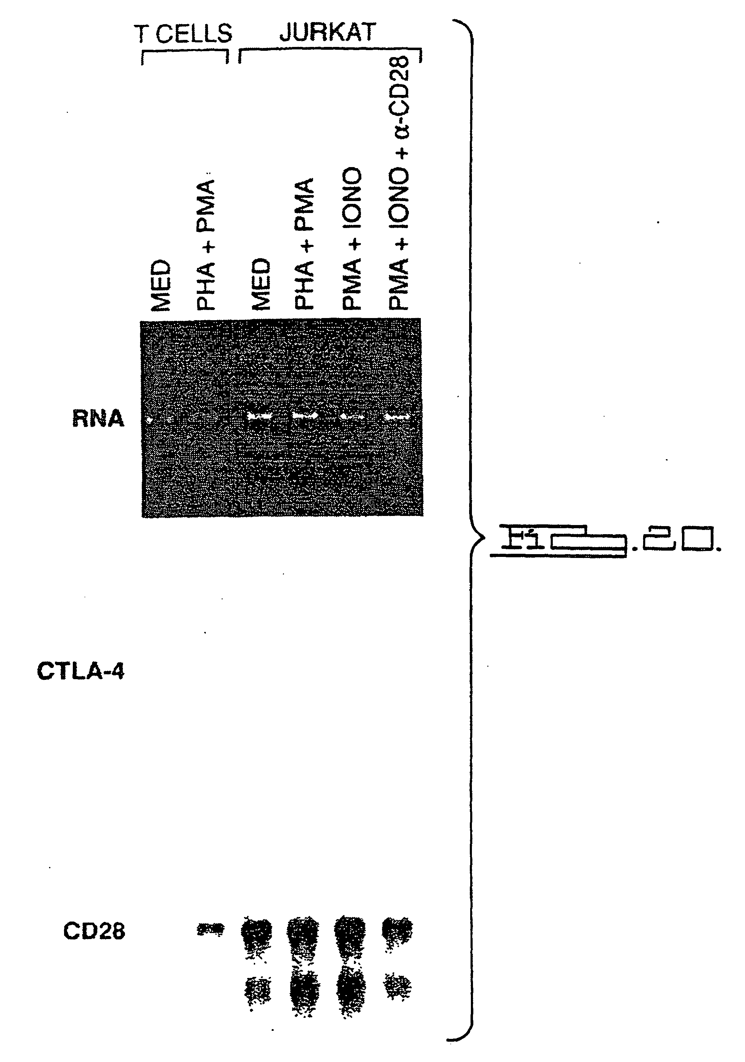

Based on the ability to show that a soluble CD28 receptor homologue, CTLA4Ig, is capable of suppressing cell-mediated responses *in vitro* and *in vivo*, experimentation was performed to determine whether or not this immunosuppressant has additive or synergistic effect with cyclosporine. A mixed lymphocyte reaction (MLR) with Brown-Norway rat lymph node cells as stimulators and Lewis strain rat lymph node cells as responders was measured by measuring tritiated thymidine incorporation 72 h after cocultivation. The ability of CTLA4Ig at a concentration of 0.1 µg/ml and cyclosporine at 30 ng/ml alone or in combination was measured. Figure 14 shows ³H-thymidine incorporation under various conditions. As can be seen in Figure 14, although either immunosuppressant led to only a partial reduction in the mixed lymphocyte proliferative response (MLR+CTLA4Ig and MLR+CSP), the combination of the two (MLR+CSP+CTLA4Ig) completely blocked the mixed lymphocyte reaction between these MHC-incompatible strains. This effect is greater than what would be expected from two immunosuppressive reagents which have additive effects, suggesting that CTLA4Ig and cyclosporine block T cell activation by independent mechanisms and have a synergistic effect on T cell activation in response to alloantigens.

### SPECIFIC EXAMPLE XI

### Control of Lymphokine Production by Second Messengers.

A variety of second messengers in the regulation of lymphokine production were examined. In particular, a role for the two primary cell secondary messenger systems, the activation of protein kinase C and elevation in intracellular calcium, were characterized as being central regulators of the transcription of tymphokine genes. In addition, specific tyrosine phosphorylation events were identified that may correlate with the generation of alterations in translation and/or mRNA stability. Further investigations into serine and threonine kinases indicate that they may also have a role in the signal transduction events involved in lymphokine production. In contrast, experiments into the regulation by cGMP showed that this agent has relatively non-specific effects on lymphokine production.

Tyrosine phosphorylation events related to CD28 were further studied as described below.

### Protocol

***Monocional antibodies*.** Anti-CD2 mAb G19-4 (IgG1), anti-CD28 mAb 9.3 (IgG2a), anti-CD5 mAb 10.2 (IgG2a), and anti-CD45 mAb 9.4 (IgG2a) were produced, purified and in some cases, biotinylated as described in Ledbetter, J.A. et al., *J. Immunol*., 135:2331 (1985) and Ledbetter, J.A. et al., *J. Immunol*., 137:3299 (1986). Anti-B7 mAb 133 (IgM) and dilutions of ascites as described in Freedman, A.S. et al., *J*. *Immunol*., 139:3260 (1987), were used. Anti-CD3 mAb OKT3 (IgG2a) was absorbed to goat anti-mouse IgG covalently linked to microspheres (KPL, Gaithersburg, MD), by incubation of a 1/10⁵ dilution of pooled ascites with 10⁷ beads/ml in HBSS at room temperature, followed by extensive washing.

***Cells*.** The CD28⁺ subset of T cells was isolated from peripheral blood T lymphocytes by negative selection using immunoabsorption with goat anti-mouse Ig-coated magnetic particles as previously described in June, C.H. et al., *Mol. Cell. Biol*., 7:4472 (1987). This resulted in a population of resting T cells that was >99% CD3⁺ and that did not contain CD2⁺/CD3⁻ cells such as NK cells. The Jurkat T leukemia cell line E6-1 was a gift from Dr. A. Weiss and maintained in complete media, i.e. RPMI 1640 containing 2 mM L-glutamine, 50 µg/ml gentamycin, and 10% FCS (HyClone Laboratories, Logan, UT). In some instances, T cells or Jurkat cells were cultured in complete media, or in complete media with 5 ng/ml PMA (Sigma Chemical Co., St. Louis, MO) or OKT3 beads (± 5 beads/cell) before experiments. The Jurkat J32 cell line (CD2⁺, CD3⁻, CD28⁺) has been described in Makni, H. et al., *J. Immunol*., 146:2522 (1991). J32 variants (CD2⁺, CD3⁻, CD28⁺) were derived by γ irradiation-induced mutagenesis and immunoselection (see Makni, *supra* (1991)); one such cloned mutant, J32-72.4 is stable in culture. The surface receptor expression of these cells was quantitated by indirect immunofluorescence and analyzed by flow cytometry. The mean log fluorescence intensity for each sample was determined and was converted into linear relative fluorescence units (ΔFL) by the formula ΔFL = 10^{[(E-} ^{C)/D]}; where *E* is the mean log fluorescence intensity of the experimental antibody sample, C is the mean log fluorescence intensity of the control antibody sample, D is 50 channels/decade. For the TCR/CD3 and CD28 receptors, ΔFL of the J32 cells was 27.0 and 57.0, and for the J32-74.2 cells 1.1 and 40.7. Northern blot analysis of J32-72.4 revealed no detectable TCR-β mRNA, while the expression of the TCR-α, CD3-γ, δ, and ε and TCR ζ mRNA was similar to that of the parental J32 cells (unpublished data).

***B7 transfection of CHO cells*.** CHO cells were transfected with B7 cDNA as previously described in Gimmi, C.D. et al., *PNAS (USA)*, 88:6575 (1991). These cells have previously been shown to stimulate lymphocyte proliferation and lymphokine secretion in a manner that mimics CD28 mAb-induced T cell activation. See Linsley, P.S. et al., *J. Exp*. *Med*., 173:721 (1991) and Gimmi, *supra* (1991). Transfected CHO cells showing no B7 expression were recloned and are referred to as CHO-B7⁻. CHO cells were detached from tissue culture plates by incubation in PBS with 0.5 mM EDTA for 30 m and fixed in 0.4% paraformaldehyde as described in Gimmi, *supra* (1991). Fixed CHO-B7⁻ cells were used as control cells.

***Immunoblot analysis of protein tyrosine phosphorylation*.** Details of the immunoblot assay with anti-phosphotyrosine antibodies has been described in Hsi, E.D. et al., *J. Biol*. *Chem*., 264:10836 (1989) and June, C.H. et al., *J. Immunol*., 144:1591 (1990). Cells were suspended at 5 - 10 x 10⁷ cells/ml in reaction media, i.e., HBSS containing 0.8% FCS and 20 mM Hepes at 37°C at time - 3 m and stimulated at time 0 m. mAbs were used at 10 µg/ml final concentration. For crosslinking, biotinylated mAbs were incubated with cells for 5 - 8 m at room temperature, the cells prewarmed at time 3 min and stimulated with avidin (Sigma Chemical Co.) at a final concentration of 40 µg/ml at time 0. Stimulation was terminated by the addition of ice-cold 10x lysis buffer, yielding a final concentration of 0.5% Triton X-100. See June, *J. Immunol*., *supra* (1990). After lysis at 4°C, nuclei were pelleted and postnuclear supernatants were subject to SDS-PAGE on a 7.5% gel, transferred to polyvinylidene difluoride microporous membrane (Millipore, Bedford, MA) and the membranes probed with affinity-purified anti-phosphotyrosine antibodies, labeled with ¹²⁵I staphylococcal protein A (ICN, Irvine, CA) and exposed to x-ray film.

### Results

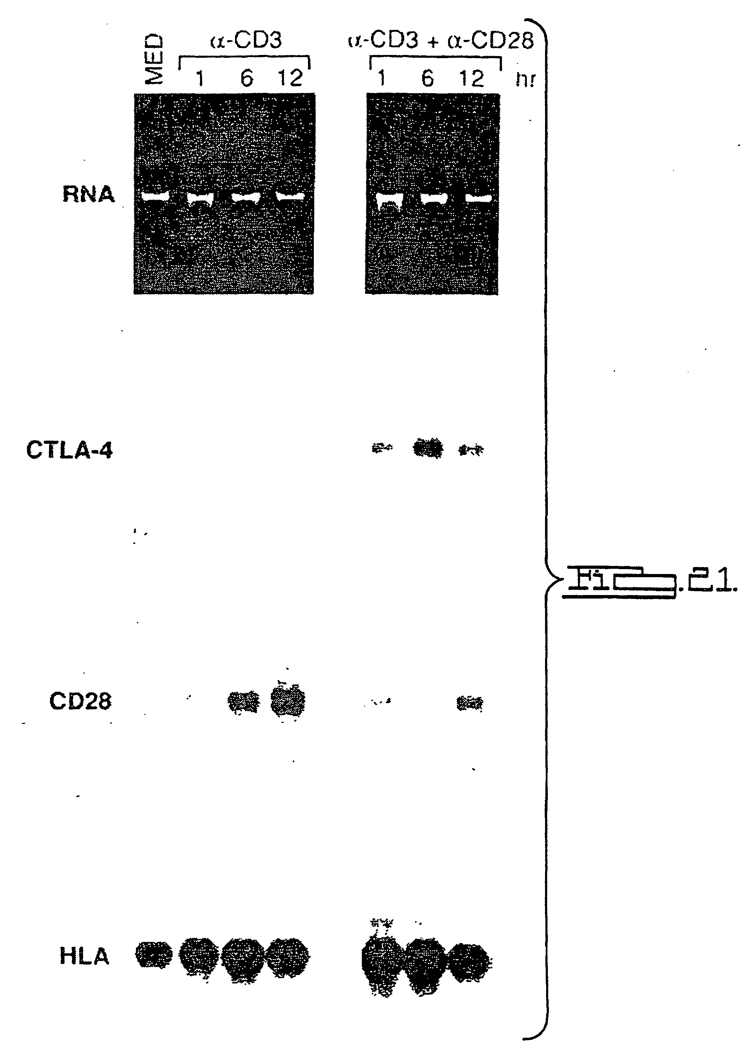

***Herbimycin A prevents CD28-stimulated IL2 production*.** Previous studies have shown that three distinct biochemical signals, provided by phorbol esters, calcium ionophore, and ligation of the CD28 receptor with mAb, are required to cause optimal IL-2 secretion (see June, C.H. et al., *J. Immunol*., 143:153 (1989)). Cells cultured in the presence of PMA, ionomycin, or CD28 mAb alone produced no detectable IL-2 and, as previously reported in June, *J. Immunol*., (1989) *supra*, and Fraser, J.D. et al., Science (Wash., D.C.) 251:313 (1991), stimulation of the CD28 receptor strongly up-regulated IL-2 production of T cells stimulated with immobilized anti-CD2 mAb, PMA, or PMA plus ionomycin. To address the potential role of tyrosine kinases in CD28-triggered signaling, the effect of herbimycin A, an inhibitor of the src family protein tyrosine kinases (see Uehara, Y. et al., *Biochem. Biophys. Res. Commun*., 163:803 (1989)), on the CD28-triggered enhancement of IL-2 production was investigated. T cells were cultured overnight in the absence (depicted as open bars in Figure 15) or presence (depicted as filled bars in Figure 15) of herbimycin A (1 µM). The cells were then cultured for a further 24 h period in the presence of medium-immobilized anti-CD3 mAb (G19-4), PMA (3ng/ml) (P), or PMA plus ionomycin (150 ng/ml) (P+I) in the presence or absence of soluble anti-CD28 mAb 9.3 (1ug/ml). Cell-free supernatant was collected, dialized to remove herbimycin A and serial dilutions were analyzed for IL-2 content by bioassay as described in June, J. *Immunol*., *supra* (1989). Figure 15 shows the effect of herbimycin A on CD28-stimulated IL-2 production. The CD28 mAb mediated enhancement of IL-2 production in response to stimulation with immobilized anti-CD3, or PMA was nearly completely inhibited in the presence of herbimycin A. In contrast, cells cultured in PMA, ionomycin or 9.3 mAb only produced < 10 U/ml of IL-2.

Disruption of the proximal signaling pathway triggered through CD3 could potentially explain the effect of herbimycin on cells stimulated with anti-CD3 and anti-CD28. Consistent with this, CD3-triggered IL-2 production was previously shown to be exquisitely sensitive to herbimycin A. See June, C.H. et al., *PNAS (USA)*, 87:7722 (1990). However, IL-2 production induced with the combination of PMA plus ionomycin or PMA plus CD28 stimulation permits, in principle, the ability to isolate the CD28 signal for testing the effect of herbimycin A. PMA plus anti-CD28-stimulated IL-2 production was sensitive to the effects of herbimycin A while, as previously noted, PMA plus ionomycin-stimulated IL-2 secretion was resistant to the effects of herbimycin A. The combination of PMA plus ionomycin plus anti-CD28-stimulation resulted in more IL-2 secretion than optimal amounts of PMA plus ionomycin, consistent with the previous reports of June, *J. Immunol*., *supra* (1989); and Fraser, J.D. et al., Science (Wash. D.C.) 251:313 (1991). However, in the presence of herbimycin A, PMA plus ionomycin plus CD28-stimulated cells produced approximately equivalent amounts of IL-2 as cells stimulated in the absence of herbimycin with PMA plus ionomycin. Together, the above results suggest that the function of both the TCR and CD28 receptors are sensitive to herbimycin, and further suggest the independent effects of these three reagents on IL-2 gene expression. See June, *J. Immunol*., *supra* (1989); and Fraser, *supra* (1991).

***CD28 receptor crosslinking with mAb induces protein tyrosine phosphorylation in PMA-treated Jurkat cells*.** Given the above functional results, the potential involvement of protein tyrosine phosphorylation in CD28-mediated signal transduction was investigated by immunoblot analysis of postnuclear supernatants of whole cell lysates of the T cell leukemia line Jurkat E6-1. Jurkat E-6 cells were cultured for 2 days in the presence or absence of PMA (5 ng/ml). After washing, 10⁷ cells in 120 µl were stimulated with reaction media (control), anti-CD3 mAb (G19-4), anti-CD28 mAb (9.3), or crosslinked anti-CD28 mAb (9.3) (final concentration, 10 µg/ml). For crosslinking, biotinylated mAb was added at time 10 m, followed by avidin (40 µg/ml) at time zero. After 2 m, the reaction was terminated with ice-cold lysis buffer and postnuclear supernatants were resolved by SDS-PAGE electrophoresis, transferred to immobilon, and immunoblotted with antiphosphotyrosine, followed by ¹²⁵I-protein A and autoradiography.

In a previous report by Ledbetter, J.A. et al., *Blood*, 75:1531 (1990), increased tyrosine phosphorylation could not be detected in resting T cells after crosslinking the CD28 receptor. Consistent with that report, no changes in tyrosine phosphorylation were detected in unstimulated Jurkat cells after the binding of bivalent or crosslinked CD28 mAb. Previous studies have shown that CD28 stimulation alone does not result in lymphokine production in Jurkat cells or induce proliferation of primary T cells. See Weiss, A. et al., *J*. *Immunol*., 137:819 (1986); Martin, P.J. et al., *J*. *Immunol*., 136:3282 (1986); and Hara, T. et al., *J. Exp. Med*., 161:1513 (1985). Engagement of CD28 by CD28 mAbs or by B7, the natural CD28 ligand, delivers a costimulatory signal provided T cells are stimulated with PMA or with TCR/CD3 mAbs. See June, C.H. et al., *Immunol*. *Today*, 11:211 (1990); Koulova, L. et al., *J*. *Exp. Med.,* 173:759 (1991); Linsley, P.S. et al., *J. Exp. Med*., 173:721 (1991); and Gimmi, C.D. et al., *PNAS (USA)* 88:6575 (1991). It thus appeared that CD28-induced protein tyrosine phosphorylation might only occur in the context of a costimulatory signal.

To test this hypothesis, Jurkat cells were cultured in PMA and then stimulated with anti-CD28 mAb as previously described. In the PMA-stimulated cells, crosslinking of CD28 for 2 m induced phosphotyrosine on substrates migrating with approximate molecular masses of 47, 62, 75, 82, 100, 110, and 145kD. Bivalent CD28 mAb induced tyrosine phosphorylation, but to a lesser magnitude. In agreement with June, C.H. et al., *J. Immunol*., 144:1591 (1990), CD3 triggering of Jurkat cells induced tyrosine phosphorylation of phosphoprotein (pp) 56, pp65, pp75, pp100, pp110, and pp145 in resting Jurkat cells and in PMA-treated Jurkat cells. Of particular interest were pp75 and pp100, which were consistently phosphorylated by CD28 stimulation under all conditions tested.

***CD28 receptor crosslinking with mAb induces protein tyrosine phosphorylation in normal T cells*.** Similar experiments with highly purified peripheral blood T cells from normal human donors were performed in order to determine if CD28 could increase tyrosine phosphorylation in nontransformed cells. Peripheral blood CD28⁺ T cells were cultured in PMA (5 ng/ml) for 6 h. After washing, 10⁷ cells were stimulated for 2 m with media (control), anti-CD3 mAb (G19.4), anti-CD28 mAb (9.3), crosslinked anti-CD28 mAb (9.3), or crosslinked anti-CD5 mAb (10.2). Cells were lysed and protein tyrosine phosphorylation was determined as previously described. Crosslinking of CD28 on PMA-treated cells induced the appearance of tyrosine phosphorylated substrates that migrated at 45, 75, and 100kD. Again, pp75 and pp100 were most prominent and consistently reproduced. The effects of CD28 stimulation observed after 24 - 48 h of PMA stimulation were more pronounced than those seen after 6 h. Ligation of CD28 by mAb on resting T cells caused the appearance of weakly detected tyrosine phosphorylation. The induction of increased responsiveness to anti-CD28 mAb stimulation by PMA is slow in that 4 - 6 h of PMA treatment are required to consistently observe CD28-induced tyrosine phosphorylation. Experiments with cycloheximide indicate that new protein synthesis is required for cells to become responsive to CD28. The specificity of the CD28-induced tyrosine phosphorylation was investigated by crosslinking CD5 with an isotype-matched mAb. Increased tyrosine phosphorylation on the 75kD substrate was occasionally induced by CD5 crosslinking. In contrast, CD5 never induced tyrosine phosphorylation on pp100. Similarly, crosslinking of the MHC class I receptor also did not induce tyrosine phosphorylation of this substrate.

***CD28 receptor crosslinking induces protein tyrosine phosphorylation In CD3-treated normal T cells.*** The above experiments suggested that the CD28 receptor is relatively inactive in quiescent cells, and becomes responsive consequent to protein kinase C activation. To determine whether TCR stimulation could also prime cells for the CD28 signal, T cells were cultured overnight in medium or in the presence of anti-CD3-coated beads. The cells were recovered, and 8 x 10⁶ cells were stimulated with crosslinked anti-CD28 mAb for 0 - 5 m, the cells lysed, and protein tyrosine phosphorylation determined as previously described. Crosslinked CD28 mAb induced low level tyrosine phosphorylation on multiple substrates in resting T cells that peaked 2 - 5 m after CD28 stimulation. In contrast, CD28 mAb induced marked tyrosine phosphorylation in CD3-primed cells that was maximal within 1 m. Thus, costimulation of T cells with anti-CD3 augmented CD28-induced tyrosine phosphorylation as manifested by an increased magnitude of response and an accelerated kinetics of response. This induction of responsiveness to CD28 did not require DNA synthesis, as separate studies have shown that the T cell blasts used for these studies were in the late G₁ phase of the cell cycle.

***CD28 receptor-B7*/*BB1 receptor interaction induces specific tyrosine phosphorylation in T cells.*** The above results indicate that CD28 mAb can increase tyrosine phosphorylation in a variety of substrates on preactivated T cells. Previous studies have indicated that CD28 appears to deliver two biochemically distinct signals, depending on the degree of crosslinking. See Ledbetter, J.A. et al., *Blood*, 75:1531 (1990). The unique functional properties of CD28 mAb observed after stimulation of T cells do not require highly crosslinked CD28 mAb and are obtained using intact or F(ab')₂ CD28 mAb. As discussed in Hara, T. et al., *J. Exp. Med*., 161:1513 (1985), studies have shown that CHO cells expressing the CD28 ligand mimic the functional effects of CD28 mAb. See Linsley, *supra* (1991), and Gimmi, *supra* (1991). These cells presumably represent a more physiologic means to study CD28 receptor-mediated signal transduction. CHO-B7⁺ cells were incubated with PMA-treated T cells at a CHO/T cell ratio of 1:10 for 5 - 30 m. B7-transfected CHO cells not expressing B7 on the cell surface (CHO-B7⁻ cells) were used as controls. Before the stimulation, CHO cells were fixed with paraformaldehyde to decrease phosphotyrosine background. Previous studies have indicated that this treatment leaves intact B7-CD28 interaction and the ensuing functional effects. See Gimmi, *supra* (1991). For the time zero point, lysis buffer was added to the T cells first, immediately followed by addition of CHO cells to the mixture. CHO-B7⁺ cells induced specific tyrosine phosphorylation that was detected primarily on a substrate that migrated at 100kD. The CHO-B7-induced tyrosine phosphorylation was detectable within 5 m of stimulation and remained elevated at plateau levels for at least 30 m. CHO-B7-induced tyrosine phosphorylation was evident at a variety of CHO-T cell ratios, and has been consistently observed for only the 100kD substrate. CHO-B7⁻ cells did not induce tyrosine phosphorylation of pp100. The B7-induced tyrosine phosphorylation was dependent upon CD28-B7 interaction as preincubation of CHO cells with anti-B7 mAb prevented CHO-B7 induced pp100 tyrosine phosphorylation. B7-CHO cells induced a slight increase in pp100 tyrosine phosphorylation in some experiments; however, this was not consistently observed.

In other experiments, alloantigen-induced T cell blasts were tested for CD28-induced tyrosine phosphorylation. T cells were culture for 8 days with allogeneic irradiated cells and then stimulated with CD28 mAb. Tyrosine phosphorylation that was most pronounced on the 74 and 100kD substrates was observed. Thus, CD28 stimulation of T cells preactivated with alloantigen, CD3 mAb, or PMA can induce tyrosine phosphorylation on a limited number of substrates that is early in onset and brief in duration.

***CD28-induced tyrosine phosphorylation prevented by CD45 and by herbimycin*.** Given that protein tyrosine kinase inhibitor herbimycin A could efficiently inhibit CD28-induced IL-2 secretion, this inhibitor was tested for effects on CD28-induced tyrosine phosphorylation. T cells were treated overnight with PMA (5 ng/ml) in the presence of the indicated concentration of herbimycin A or in control medium. The cells were collected, washed, and 8 x 10⁶ cells were stimulated with media or with crosslinked anti-CD28 mAb for 2 m. Detergent-soluble proteins were processed as previously described. Tyrosine phosphorylation induced by anti-CD28 mAb was nearly completely prevented in herbimycin-treated cells under conditions that specifically inhibit CD28-induced IL-2 production.

The brief temporal course of CD28 mAb-induced tyrosine phosphorylation suggested regulation by a phosphatase. To address the effects of phosphatases on CD28-mediated signal transduction, T cells were cultured overnight with PMA (5 ng/ml). 10⁷ cells were incubated for 10 m with media (control), biotinylated anti-CD45 mAb (9.4), anti-CD28 mAb (9.3), or both. Monoclonal antibodies were crosslinked with avidin at time 0. The reaction was terminated after 2 m. Immunoblot analysis with antiphosphotyrosine antibodies of detergent-soluble proteins was performed as previously described. CD28 crosslinking induced tyrosine phosphorylation on pp75 and pp100 that was completely prevented by CD45. Consistent with previous results described in Samelson, L.E. et al., *J. Immunol*., 145:2448 (1990), crosslinking of CD45 alone caused increased tyrosine phosphorylation of a 120-135kD substrate; this effect is also seen in CD28 plus CD45-treated cells. Thus, the above studies indicate that CD28-tnduced tyrosine phosphorylation is sensitive to an inhibitor of src family protein tyrosine kinases, and furthermore, that the CD45 protein tyrosine phosphatase can prevent CD28-induced protein tyrosine phosphorylation.

***CTLA-4 expression predicts expression of IL-2 following CD28 pathway activation*.** Purified resting T cells were stimulated with immobilized anti-CD2 Ab, anti-CD3 + mAb 9.3, PMA + ionomycin, PMA + mAb 9.3 and PMA + ionomycin + mAb 9.3 in the presence or absence of the protein-tyrosine kinase inhibitor herbimycin for 8 h. Duplicate Northern blots were hybridized to CTLA-4, CD28, IL-2 or HLA specific probes. Expression of CD28, CTLA-4 and IL-2 was then analyzed by Northern blot. IL-2 expression correlated well with CTLA-4 expression following CD28 pathway activation. CTLA-4 and IL-2 expression were also suppressed to a similar degree with herbimycin while CD28 expression remained unchanged. This suggests that the suppressive effects of protein-tyrosine kinase inhibitors on CD28 pathway activation may be mediated through suppression of CTLA-4 expression.

### SPECIFIC EXAMPLE XII

### Induction of MHC Independent T Cell Proliferation by CD28 and Staphylococcus Enterotoxins.

### Protocol

***Isolation of T cells.*** Peripheral blood was drawn from normal human volunteers. The mononuclear cell fraction was obtained by density gradient centrifugation through a Ficoll-Hypaque (Pharmacia) cushion. This fraction was used in experiments utilizing peripheral blood mononuclear cells (PBMCs). Purified resting T cells were obtained by incubating the mononuclear cells with an antibody cocktail directed against B cells, monocytes and activated T cells. The antibody coated cells were then removed by incubation with goat anti-mouse immunoglobulin-coated magnetic beads (Advanced Magnetics Inc.) as previously described in June, *supra* (1987). This method has routinely yielded a population > 99% CD2⁺ by flow cytometry.

***Proliferation assays.*** Proliferation was measured by culturing 5 x 10⁵ purified T cells or PBMC's in each well of a 96 well microtiter plate. The final culture volume was 200 µl of RPMI 1640 (Gibco) supplemented with 10% FCS, penicillin (100 U/ml), streptomycin (100 µg/ml) and 2 mM L-glutamine. Staphylococcal Enterotoxin A (SEA), Staphylococcal Enterotoxin B (SEB) (Toxin Technologies) and cyclosporine A (Sandoz) were added in the indicated doses at the initiation of the culture. Anti-CD28 monoclonal antibody (mAb 9.3, gift from J. Ledbetter) and anti-HLA-DR monoclonal antibody (mAb L243, gift from J. Ledbetter) were added at the start of the culture period. Tritiated thymidine (³H-TdR, ICN) was included at a concentration of 1 µCi per well for the final 8 h of the culture. The cells were harvested onto glass microfiber filter strips (Whatman) after 72 h using a PHD cell harvester (Cambridge Technologies) and counted on a liquid scintillation counter (LKB). All values are expressed as the mean cpm ± standard deviation of triplicate or quadruplicate cultures.

***Flow Cytometry***. One ml cultures of T cells were incubated with media alone, SEA (100 ng/ml) or SEB (1 µg/ml), SEA or SEB plus anti-CD28 antibody (1 µg/ml) or PMA (3 ng/ml) plus anti-CD28 antibody (1 µg/ml) at 37°C for 72 h. Aliquots of each sample were stained with acridine orange (Polysciences) for cell cycle analysis as described in Darzynkiewicz, Z., *Meth. Cell. Biol*., 33:285 (1990), FITC conjugated anti-IL2 receptor antibody (Coulter), FITC conjugated anti-HLA-DR antibody (Becton-Dickinson) or an isotype-matched irrelevant antibody (Becton-Dickinson). Each sample was analyzed on a FACScan flow cytometer (Becton-Dickinson).

### Results

***CD28 provides costimulatory activity for superantigen-activated purified T cells*.** Highly purified T cells were cultured with graded concentrations of either SEA (0.1 ng/ml to 1.0 µg/ml) or SEB (.01 µg/ml to 100 µg/ml). Replicate cultures were prepared in which a stimulatory antibody to CD28 was added. The cultures were pulsed with ³H-TdR for the final 8 h of a 72 h culture and incorporated thymidine determined by liquid scintillation counting as described above. Each condition was performed in quadruplicate. Treatment with SEB alone failed to induce thymidine incorporation above control cultures. However, the addition of anti-CD28 antibody resulted in significant proliferation to graded doses of SEB. Treatment with anti-CD28 antibody alone had no effect. The lack of accessory cells was verified by an absence of proliferation to PHA. Identical results were obtained using SEA.

***Stimulation with SEA or SEB leads to cell cycle entry*.** Since CD28 stimulation alone does not induce T cell cycle entry, the observation that CD28 provided costimulatory activity for T cells treated with either SEA or SEB suggested that these enterotoxins could induce cell cycle entry in purified T cells. In order to examine this, purified T cell cultures were stimulated with SEB (1 µg/ml) alone or with SEB (1 µg/ml) plus anti-CD28 monoclonal antibody (1 µg/ml) for 48 h and stained with acridine orange for cell cycle analysis. Unstimulated cells were run simultaneously in order to determine the G₀/G₁ interface. Those with an increased RNA content but unchanged DNA content were considered G₁ phase cells. Cells with increases in both RNA and DNA content were considered in S, G₂ or M phases. Concomitantly, aliquots were stained with FITC-conjugated anti-IL-2 receptor antibody. Treatment with enterotoxin alone for 48 h resulted in progression of greater than 10% of the T cells from G₀ to G₁ as determined by an increase in RNA staining with no increase in DNA content. Similarly, enterotoxin alone induced IL-2 receptor expression in 15% of the cells at 72 h. In contrast, when the anti-CD28 monoclonal antibody was present, a significant proportion of the cells that had left the G₀ stage of the cell cycle were found to have increased their DNA content and thus are in either the S, G₂ or M phases of the cell cycle. These data indicate that stimulation with SEB alone is sufficient to activate the T cell but delivers an inadequate signal for complete progression through the cell cycle. Provision of a second signal by simultaneous stimulation of the CD28 pathway allowed the cell to progress to S-phase and proliferate.

***Proliferation of T cells stimulated by SEA and anti-CD28 is resistant to cyclosporine A*.** CD28 has been shown to utilize a signal transduction pathway that is resistant to the effects of cyclosporine A (CsA) when the initial signal is provided by PMA, and partially resistant to CsA when cells are initially activated through the T cell receptor. See June, *supra* (1987). In order to further examine the pathways involved in T cell activation by enterotoxin, cyclosporine A (1 µg/ml) was included in cultures activated by SEA and SEA plus anti-CD28 antibody (³H-TdR incorporation determined as described above). As for SEB, SEA alone did not induce thymidine incorporation whereas addition of antibody against CD28 resulted in significant proliferation. Even in the presence of cyclosporine A (1 µg/ml), there was a dose dependent increase in proliferation when cultures were activated by a combination of SEA and anti-CD28 antibody. Control cultures activated with PMA plus anti-CD28 antibody were resistant to cyclosporine A and activation by PMA plus ionomycin was sensitive to cyclosporine A.

***Activation by SEB and anti-CD28 is independent of class II MHC*.** Previous work has demonstrated that the staphylococcal enterotoxins are capable of simultaneously binding the TCR and class II MHC molecules on the surface of antigen presenting cells (APCs). See Herrmann, T. et al., *Eur. J. Immunol*., 19:2171 (1989); and Chintagumpala, M.M. et al., *J*. *Immunol*., 147:3876 (1991). The observation that proliferation was not observed unless APCs were present in the culture was interpreted to mean that T cell activation by superantigen is dependent upon class II MHC expression as discussed in Fleischer, B. et al., *J. Exp. Med*., 167:1697 (1988); Carisson, R. et al., *J. Immunol*., 140:2484 (1988); and Herman, A. et al., *J. Exp. Med*., 172:709 (1990). Our observation that highly purified T cells could be induced to proliferate by simultaneous stimulation with enterotoxin and anti-CD28 antibody suggested that class II MHC may not be absolutely required for superantigen activation of T cells. Alternatively, activated T cells can express class II MHC and thus might provide class II-dependent superantigen presentation to other T cells in trans.

To examine this possibility, a blocking antibody against HLA-DR, monoclonal antibody L243, was included in cultures of T cells and PBMCs activated by enterotoxin or enterotoxin plus anti-CD28 antibody as shown in Figure 16. Each point is expressed as the mean ± the standard deviation of triplicate or quadruplicate cultures. No significant proliferation was observed with SEB alone. As shown previously, inclusion of anti-CD28 antibody allows SEB to induce T cell proliferation in a dose-dependent manner. There was no decrease in proliferation when anti-class II antibody was included in the cultures at doses of 1.0 or 10 µg/ml (data for 10 µg/ml not shown). In contrast, as shown in Figure 17, the proliferation of PBMCs isolated from the same donor and stimulated with enterotoxin was significantly inhibited by anti-class II antibody. In addition, HLA-DR expression at 24 and 72 h was examined by purified T cells activated with SEA (0.1 or 1.0 ng/ml) with and without CD28 costimulation. There was no expression of HLA-DR in either condition as determined by flow cytometry. This indicated that the T cell proliferation induced by enterotoxin + anti-CD28 is not dependent on presentation by an MHC class II molecule.

### SPECIFIC EXAMPLE XIII

### Prevention of Programmed Cell Death.

A series of experiments were done to test whether anti-CD28 mAb might prevent cell death in mature T cells. Jurkat leukemia cells are commonly used as an example of mature T cells that mimic physiologic effects found in peripheral blood T cells. For example, Jurkat cells can be induced to secrete IL-2 with anti-CD3 mAb and anti-CD28 mAb, and Jurkat cells can be infected and killed by HIV-1. The Jurkat line JHMI-2.2 was obtained from A. Weiss (UCSF); the muscarinic M₁ receptor subtype has been transfected and is stably expressed in these cells. JHMI-2.2 cells, 0.3 x 10⁶/well, were added to culture wells in complete medium, or to wells that contained plastic-adsorbed anti-CD3 mAb G19-4, in the presence or absence of 9.3 mAb 10 µg/ml, or 9.3 mAb alone. Cell death was scored after 1 to 3 days of culture and graded as 0 (none), 1 + (20 to 70% of cells dead), and 2+ (70 to 100% of cells dead), and was determined by visual inspection of the wells, and confirmed by trypan blue permeability. As shown in Table 10, cells in medium continued to grow and remain viable while cells in anti-CD3-treated wells died. In contrast, the cells in wells containing anti-CD3 plus anti-CD28 continued to proliferate. The ability of anti-CD28 to rescue cells from anti-CD3-induced cell death was specific, because carbacol (30 µM) (a specific agonist of M₁ receptors that is believed to activate signal transduction in cells via a mechanism distinct from the T cell receptor/CD3 complex), also induced cell death in Jurkat cells. Anti-CD28 did not prevent carbacol-induced cell death.

**TABLE 10**

| CONDITION | CELL DEATH (GRADE 0-2) |
|---|---|
| EXPERIMENT #1 | |
| Medium | 0 |
| anti-CD3 | 2+ |
| anti-CD3 + anti-CD28 | 0 |

| EXPERIMENT #2 | |
|---|---|
| Medium | 0 |
| Carbacol | 1+ |
| Carbacol + anti-CD28 | 2+ |

### SPECIFIC EXAMPLE XIV

### Bone Marrow Studies.

***Proliferation of T cells after activation of T cells with soluble or immobilized anti-CD3 (OK73)*.** A series of titration studies were performed using soluble or immobilized OKT3 to activate and induce T cell proliferation. Immobilized OKT3 (2 µg/ml precoated plates for 1 h at 37°C) and soluble OKT3 (10 ng/ml) consistently induced T cell proliferative responses from E-rosette (E⁺) purified T cells or PBL PBL or purified T cells were activated by incubation for 1 h to 7 days on immobilized OKT3 or by adding 10 ng/ml of soluble OKT3 at the beginning of culture. In a series of experiments, proliferation after activation of T cells with immobilized OKT3 was comparable to proliferative responses by PBL after activation with soluble OKT3.

***Cytotoxicity mediated by anti-CD3 and anti-CD28 triggered PBL***. The cytotoxicity of anti-CD3 activated PBL after 7 days of culture in the presence of low doses of IL-2 or anti-CD28 was tested. In this set of experiments, the ability of CD28 to induce increases in lymphokine production to substitute for previously reported immune augmented effects of *in vitro* T cell treatment with IL-2 has been examined. One lytic unit is equivalent to 20% lysis of 5 x 10³ target cells per 1 x 10⁶ effector cells as discussed in Press, H.F. et al., *J. Clin. Immunol*. 1:51-83 (1981). Various targets, including Daudi and K562, were tested. Cytotoxicity results of a representative experiment are shown in Table 11.

**TABLE 11**

| CELL GROWTH STIMULUS | Target - Daudi (LU) | Target - K562 (LU) |
|---|---|---|
| anti-CD3 plus IL-2 | 14.9 | 10.6 |
| anti-CD3 plus anti-CD28 | 12.1 | 6.1 |

***OKT3-induced cytotoxicity in PBL comparable to T cells*.** In 5 different subjects, PBL were compared with T cells (E⁺) in their ability to kill Daudi, K562, and BSB cells 8 days after being activated with OKT3. These experiments were performed in X-Vivo 10 supplemented with 5% human serum (HS). The mean cytotoxicity in 5 normal subjects using PBL directed at Daudi, K562, and BSB were 15.0, 7.4, and 9.2 LU, respectively. In T cells from the same 5 subjects, the mean cytotoxicity directed at Daudi, K562, and BSB were 14.3, 7.7, and 9.3 LU, respectively.

***Cytotoxicity as a function of in vitro time in cell culture.*** In order to test for optimal cytotoxicity, T cells were cultured with anti-CD3 and IL-2 for 31 days and tested at weekly intervals for cytotoxicity against Daudi and K562. Logarithmic cell growth was maintained during this time, with a 300-fold expansion in cell number. Cytotoxicity was a strong function of culture duration however, with <0.1, 24, 3.5, 0.5, 1.0 LU at 0, 8, 15, 22, and 29 days of culture. Similar results were found when Daudi was the target, with <.01, 23, 5, 2, and 5 LU at 0, 8, 15, 22, and 29 days of culture.

***Cytotoxicity induced by soluble or immobilized OKT3*.** In 7 experiments, cytotoxicity mediated by T cells after activation was compared with soluble or immobilized OKT3. Both methods induced cytotoxicity directed at Daudi and K562. Soluble OKT3 activated T cells mediated a mean cytotoxicity of 27 LU (SD - 18) directed at Daudi and a mean cytotoxicity of 21 LU (SD - 16) directed at K562. Immobilized OKT3 activated T cells mediated a mean cytotoxicity of 22 LU (SD - 16) directed at Daudi and a mean cytotoxicity of 12 LU (SD - 8) directed at K562. These experiments were performed with E⁺ cells in RPMI 1640 supplemented with 10% fetal bovine serum (FBS). Cytotoxicity was assessed 7 to 8 days after triggering with soluble (10 ng/ml) or immobilized (2 µg/ml) OKT3.

***Effects of IL-2 concentrations*.** The dose of IL-2 was titrated after establishing the optimal time of OKT3 activation. In several experiments, the doses of IL-2 were gradually reduced from 6000 IU/ml to 60 IU/ml. Proliferation, as measured by tritiated thymidine incorporation after 3 days of culture, remained constant as IL-2 was titrated in this range. In contrast, cytotoxicity varied with the dose of IL-2, and was maximal at lower doses of IL-2 (lytic units with Daudi targets were 28, 9, 8.5, 9 and 4.8 LU after culture in 30, 150, 300, 600 and 6000 IU/ml of rIL-2). The data show that both proliferative and cytotoxic responses of the anti-CD3 triggered T cells can be obtained and maintained in low doses of IL-2.

***Effects of serum and medium on proliferation and cytotoxicity*.** The ability of HS, FBS, and serum free media were compared for their ability to support growth and maintain cytotoxicity. The data show that proliferation and cytotoxicity directed at Daudi, K562, and BSB rapidly decreased below a serum concentration of 2%. There were no significant differences between X-Vivo 10 and RPMI 1640.

***Bone marrow mononuclear cells (BMMNC) as a source of CTC after anti-CD3 and anti-CD28 treatment***. To test whether BMMNC might serve as a source of T cells for therapy in patients with malignancies, BMMNC were cultured in X-Vivo 10 medium after OKT3 and IL-2 or anti-CD28 stimulation. Although the BMMNC population initially contained only 25% CD3⁺ cells, proliferative and cytotoxic responses were excellent after 2 weeks of culture. T cells expanded more than 40-fold after CD3 and IL-2 stimulation and cytotoxicity was between 5 and 12 LU at 8 to 15 days of culture when tested against Daudi and K562. These data show that BMMNC obtained from autologous bone marrow harvest from a patient before bone marrow transplantation provide a suitable source of cytotoxic T cells. Table 12 shows that both normal and patient bone marrows provide satisfactory sources of cytotoxicity after CD3 and IL-2 treatment. In 4 experiments using normal bone marrow or autologous bone marrow, there was a median of 89-fold expansion of cells (range 18-to 173-fold) after 9 to 19 days of culture. Mean cytotoxicity directed at Daudi and K562 mediated by BMMNC stimulated with OKT3 was 5.5 LU (range 2 - 11) and 4.3 LU (range 2 - 8), respectively. All cultures were tested 14 days after activation with OKT3 and expanded in the presence of 50 IU/ml of IL-2. Either soluble OKT3 10ng/ml(s) or immobilized OKT3 (coated with 2 µg/ml)(I) were added as indicated.

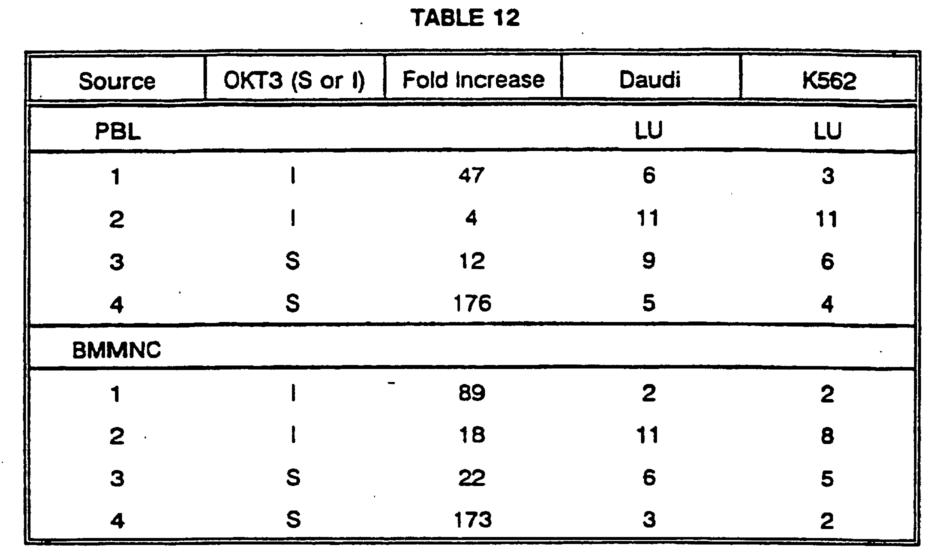

***Anti-CD3 plus anti-CD28 treatment of BMMNC as a source of effector T cells*.** Proliferative and cytotoxic responses from 3 patients were tested. The patients had received extensive chemotherapy and yet their PBL or BMMNC maintained strong proliferative and cytotoxic responses after anti-CD3 plus anti-CD28 treatment. PBL or BMMNC (1.5 X 10⁵) were cultured in RPMI plus 5% human serum in the presence of immobilized OKT3 mAb or 50 IU/ml rIL-2 or 9.3 mAb 0.5 µg/ml. Proliferation was assessed on day 3 of culture and cytotoxicity on day 7. Table 13 summarizes the results for BMMNC.

**TABLE 13**

| SAMPLE | Proliferation ³H incorp. (cpm) | Cytotoxicity Daudi (LU) | Cytotoxicity K562 (LU) |
|---|---|---|---|
| Bone Marrow #171 AML, Relapsed | | | |
| OKT3 | 23,479 | - | - |
| OKT3 + anti-CD28 mAb | 58,563 | 18.0 | 4.8 |
| OKT3 + IL-2 | 48,745 | 15.1 | 4.6 |
| PBL P#632 non-Hodgkins lymphoma, pre-transplant | | | |
| OKT3 | 58,670 | - | - |
| OKT3 + anti-CD28 mAb | 77,046 | 14.8 | 12.7 |
| OKT3 + IL-2 | 63,603 | 18.8 | 18.1 |
| PBL P#635 Hodgkins lymphoma, pre-transplant | | | |
| OKT3 | 27,758 | - | - |
| OKT3 + anti-CD28 mAb | 46,133 | - | - |
| OKT3 + IL-2 | 43,918 | - | - |

***OKT3-activated T cells (CTC) do not inhibit hematopoietic progenitor growth.*** In order to determine whether BMMNC mixed with OKT3-activated T cells (CTC) in hematopoietic progenitor assays would inhibit the development of CFU-GM, CTC obtained from PBL after a week of growth was mixed with fresh BMMNC and plated the mixtures into the CFU-GM assay. The autologous CTC were mixed with BMMNC in various ratios, incubated for 1 h at 37°, and then plated in a standard CFU-GM assay. The CTC had no deleterious effect on colony formation, as the number of CFU-GM colonies was within 75% of control over a wide variety CTC:BMMNC ratios (ratios of 1:25 to 5). The number of CFU-GM colonies was not inhibited greater than 90% (an accepted % inhibition of CFU-GM in purged autologous marrow grafts) even at a ratio of 1 CTC to 1 BMMNC. These data suggest that CTC will not inhibit or delay engraftment of autologous bone marrow transplants. /

***Expansion and cytotoxic functions of PBL or BMMNC from patients before BMT*.** In order to determine whether PBL or BMMNC from patients heavily pretreated for AML or lymphoma could be activated with anti-CD3 and grown in low dose IL-2, PBL or BMMNC obtained prior to BMT on several patients was tested. The PBL and BMMNC of the patients tested proliferated and exhibited cytoxicity in a fashion comparable to that seen in normal PBL or BMMNC obtained from normal allogeneic marrow donors. It was anticipated that some patients that had been heavily treated with chemotherapy or radiation would have low counts or have poor responses to anti-CD3 activation. Thus, the number of starting cells was increased in the protocol to compensate for cell loss or inability to proliferate. The use of 9.3 as a costimulant to anti-CD3 activated T cells to enhance helper activity or enhance cytotoxicity could result in improved *in vitro* expansion of activated T cells. Data presented in this example (Specific Example XIV) show that mAb 9.3 can correct proliferative defects in post-transplant lymphocytes further supporting the rationale for using OKT3/9.3 costimulation approach to accelerate immune reconstitution and enhance cytotoxicity directed at mallignant cells in ABMT recipients. A recent study by Katsanis, E. et al., *Blood* 78:1286-1291 (1991) shows that T cells from BMT recipients can be expanded by stimulation with OKT3 and IL-2.

***Messenger RNA levels for IL-2 receptors (IL-2R), IL-2*, *and IL-3 in PBL from short and long-term BMT recipients.*** Earlier studies (see Lum, L.G. et al., *Blood Suppl*. (Abstract) (1991)) suggested that T cells from BMT recipients fail to secrete IL-2 or express IL-2R. Such defects may be due to failure of mRNA synthesis for lymphokines or lymphokine receptors. A determination was made whether T cells from BMT patients failed to express detectable levels of mRNA for IL-2, IL-2R and IL-3. PBL from 11 allogeneic (3 short-term, ST, and 8 long-term, LT) and 4 autologous recipients (2 ST and 2 LT) were tested for levels of IL-2R, IL-2, and IL-3 mRNAs without stimulation (-) or after phytohemagglutinin (PHA) and phorbol ester (TPA) stimulation (+). cDNA synthesized by reverse transcriptase (RTase) from total RNA was amplified by PCR using specific primers and the PCR products run on 1.5% agarose gels containing ethidium bromide. Table 14 shows the fraction and percent of recipients whose PBL had detectable levels of mRNA for IL-2R, IL-2, and IL-3.

**TABLE 14**

| STIMULATION | IL-2R (%) | IL-2 (%) | IL-3 (%) |
|---|---|---|---|
| Allogeneic Recipients | | | |
| - | 2/11 (18) | 1/9 (11) | 8/11 (73) |
| + | 9/11 (82) | 8/9 (89) | 7/10 (70) |

| Autologous Recipients | | | |
|---|---|---|---|
| - | 2/4 (50) | 3/4 (75) | 4/4 (100) |
| + | 4/4 (100) | 3/4 (75) | 3/4 (75) |

PBL from a high proportion of ST and LT autologous and allogeneic BMT recipients expressed levels of mRNAs for IL-2R, IL-2, and IL-3 after stimulation with PHA+TPA. In the ST recipients tested, 2 of 2 ABMT recipients and 3 of 3 allogeneic recipients tested had PBL that expressed mRNA levels of IL-2R and IL-3; 2 of 2 allogeneic recipients tested expressed mRNA for IL-2. In most cases, defective mRNA synthesis for IL-2R, IL-2, and IL-3 may not be responsible for defects in IL-2 secretion and IL-2R expression. Posttranscriptional events may play a more important role in defective lymphokine secretion by T cells from BMT recipients.

***CTC help Ig synthesis and express mRNA for lymphokines and perforin*.** As discussed in Ueda, M. et al., *J. Cell. Biochem.* (Abstract) (submitted 1992), helper activity was assessed by adding normal T cells or T cells activated with OKT3 to normal B cells after PW stimulation as measured by an ELISA-Plaque (PFC) assay. The number of PFC per million B cells cultured was 3200, 4100, 8800 when 25, 50 or 75 X 10³ normal T cells were added. When the same numbers of CTC were added, the number of PFC were 220, 2100, and 2600. Thus, CTC exhibit substantial helper activity. Furthermore, CTC did not suppress normal autologous or allogeneic T and B cells in a suppressor assay for Ig synthesis. Helper activity was radioresistant. Messenger RNA for IL-2, IL-3, IL-6 and perforin was detected from 6 h to more than 3 days after OKT3 activation using a RTase-PCR method. In summary, CTC help B cells produced Ig and did not suppress Ig synthesis by normal T and B cells. Thus, adoptive transfer of CTC after BMT may not only mediate a GVL effect but may accelerate immune reconstitution.

***Defects in anti-CD3-induced proliferative responses in PBL from BMT patients repaired by adding anti-CD28*.** *In vitro* data on anti-CD3 and anti-CD3/anti-CD28 stimulated proliferative responses of T cells from BMT recipients support the premise that using mAb 9.3 in combination with OKT3 may have potent *in vivo* clinical effects as reported. See Joshi, I. et al., *Blood Suppl*. (Abstract) (1991). T cells from BMT recipients have defects in proliferation after mitogen or anti-CD3 stimulation. Previous studies show that costimulation of normal T cells with anti-CD3 (G19-4) and 9.3 enhance anti-CD3-induced proliferation by stabilizing lymphokine mRNAs. Experimentation to assess the ability of anti-CD3 (G19-4 or OKT3) + 9.3 to correct defective anti-CD3-induced proliferative responses in PBL from autologous and allogeneic BMT recipients (53-605 days post BMT) was performed. PBL from recipients or controls were stimulated for 3 days with G19-4, G19-4+9.3, OKT3, or OKT3+9.3. 9.3 was added at a final concentration of 100 ng/ml. Fifteen tests were performed on ABMT recipients and sixteen tests were performed on allogeneic recipients. Table 15 shows the number of recipients whose PBL increased (↑), or decreased (↓), or did not change (⇔) their proliferative responses after the addition of 9.3 to anti-CD3 stimulated PBL The parenthesis indicate percent of recipients whose proliferative responses increased after the addition of 9.3.

**TABLE 15**

| BTM RECIPIENTS | CHANGE | TREATMENT | |
|---|---|---|---|
| | | G19-4+9.3 | OKT3+9.3 |
| | ↑ | 9 (60%) | 9 (82%) |
| Autologous BMT | ↓ | 3 (20%) | 2 (18%) |
| | ⇔ | 3 (20%) | 0 (0%) |
| | ↑ | 11 (69%) | 8 (62%) |
| Allogeneic BMT | ↓ | 5 (31%) | 5 (38%) |
| | ⇔ | 0 (0%) | 0 (0%) |

Costimulation of G19-4+9.3 or OKT3+9.3 significantly increased proliferative responses induced by G19-4 or OKT3 alone (p < 0.05, paired rank-sum) in ABMT recipients. In summary, defects in anti-CD3-induced T cell proliferation in BMT recipients were repaired by costimulation with 9.3. These findings have therapeutic implications for patients with immune defects manifest with impaired T cell proliferation. Indeed similar results have been obtained which indicate that the proliferative defect of T cells from patients with HIV infection can be repaired with anti-CD28 treatment. See Lane, H.C. et al., *J. Engl*. *J. Med*., 313:85 (1985) regarding proliferate defects in HIV.

***Costimulation with anti-CD3 OKT3 and anti-CD28 9.3 enhanced detectable mRNA levels for IL-2 in PBL from ABMT recipient*.** PBL from a short-term ABMT recipient were studied for expression of mRNA levels for IL-2 after stimulation with OKT3 and costimulation with OKT3/9.3 using RTase-PCR. cDNA synthesized by RTase from total RNA was amplified by PCR using specific primers for IL-2 and the PCR products run on 1.5% agarose gels containing ethidium bromide. Consistent with the findings in the previous paragraph, activated T cells from the ABMT recipient did not have detectable levels of mRNA for IL-2 after OKT3 stimulation alone, whereas the same T cells costimulated with OKT3/9.3 had a distinct band for IL-2 of 458 bp detected on ethidium bromide stained agarose gel. This is an example of how OKT3/9.3 costimulation can repair an apparent defect in the expression of mRNA for IL-2 in T cells from ABMT recipients.

***Stimulation of negatively selected CD4***^{***+***} ***cells with anti-CD28 9.3 after activation with anti-CD3 induces IL-2 independent proliferative responses.*** CD4⁺ cells were purified by a series of negative selection steps as previously described in Thompson, C.B. et al., *PNAS (USA)* 86:1333-1337 (1989). PBL were incubated with a cocktail of mAbs directed at non-CD28⁺ cells, washed, and incubated with immunomagnetic bead coated with goat anti-mouse antibody. The CD28⁺ enriched cells were further purified by removing the CD8⁺ cells by treatment with anti-CD8 and binding the CD8⁺ cells to the immunomagnetic beads.

The remaining CD28⁺, CD4⁺ T cells from a normal donor were cultured by adding cells to culture dishes containing plastic adsorbed OKT3. After 48 h, the cells were removed and placed in flasks containing either rIL-2 (200 IU/ml) or anti-CD28 mAb (100 ng/ml). The cells were fed with fresh medium as required to maintain a cell density of 0.5 X 10⁶/ml, and restimulated at approximately weekly intervals by culture on plastic adsorbed OKT3 for 24 h. The cells could be maintained in logarithmic growth, with a 4 to 5 log₁₀ expansion in cells number. As shown in Figure 18, cells propagated with anti-CD3 and anti-CD28 routinely expanded 10 to 30-fold more than cells grown in optimal amounts of anti-CD3 and IL-2. When synthetic medium (X-Vivo 10) not containing FBS was used, anti-CD3 plus anti-CD28 treated cells also expanded 10-fold better than anti-CD3 plus IL-2 treated cells. The highly enriched CD4 cells did not proliferate in the presence of optimal amounts of the lectin phytohemagglutinin (PHA). Thus, the *in vitro* expansion of CD4 cells using anti-CD28 has an advantage over previously described methods, in that it is independent of the addition of exogenous growth factors, as no IL-2 or any other growth factors were added to these cells. In addition, this system does not require the presence of accessory cells, which is advantageous in clinical situations where accessory cells are limiting or defective.

***Phenotypes of anti-CD3 activated T cells.*** Populations of CTC cells grown in IL-2 for 6 to 12 days contained predominantly CD3⁺ cells (greater than 84%, median 88%). The proportion of CD56⁺ cells (a marker for NK cells) was less than 1.3%. Triggering of E⁺cells with OKT3 is preferentially selecting CD3⁺ cells. CD4⁺ cells were 18% or less and CD8⁺ cells were greater than 66%. Lytic activity did not correlate with the proportions of CD56⁺ cells in the cultures.

***Immunophenotype of T cells differs after anti-CD28 and IL-2-mediated cellular growth.*** To examine the subsets of T cells that are expanded, PBL were propagated for 16 days using either anti-CD3 and IL-2 or anti-CD3 and anti-CD28. The percentage of CD4 and CD8 cells was 23.8 and 84.5 in the cells grown in IL-2, and 56.0 and 52.6 in the cells grown in CD28. These results suggests that CD28 expansion favors the CD4+ cells, in contrast to the well established observation that CD8⁺ cells predominate in cells grown in IL-2 (for example, see above paragraph; see also Cantrell, D.A. et al., *J. Exp. Med.* 158:1895 (1983)). To further test this possibility, CD4 cells were enriched to 98% purity using negative selection with monoclonal antibodies and magnetic immunobeads as described elsewhere in this example. The cells were cultured for one month using anti-CD3 and either IL-2 or anti-CD28 to propagate the cells. There was equal expansion of the cells for the first 26 days of the culture, however, as can be seen in Table 16, the phenotype of cells diverged progressively with increasing time in culture.

**TABLE 16**

| CULTURE METHOD | DAYS | CD3 (%) | CD4 (%) | CD8 (%) |
|---|---|---|---|---|
| anti-CD3 + IL-2 | 0 | >99 | >99 | <1 |
| | 6 | >99 | 98 | 1 |
| | 12 | >99 | 85 | 10 |
| | 20 | >99 | 45 | 40 |
| | 26 | >99 | 12 | 78 |
| anti-CD3 + IL-2 | 0 | >99 | >99 | <1 |
| | 6 | >99 | >99 | <1 |
| | 12 | >99 | >99 | <1 |
| | 20 | >99 | >99 | <1 |
| | 26 | >99 | >99 | <1 |

***Use of anti-CD28 for in vitro expansion of TIL***. The use of IL-2 and inactivated tumor cells to expand tumor infiltrating tymphocytes (TIL cells) for later adoptive immunotherapy with a variety of neoplasms has demonstrated promise (*e*.*g*., see Rosenberg, S.A. et al., *NEJM* 323:570-578 (1990)). TIL cells were isolated from a nephrectomy specimen from a patient with renal cell carcinoma. The cells were cultured with tumor cells, and either IL-2 or anti-CD28 and IL-2 with mAb OKT3 added at weekly intervals, beginning at day 14. Table 17 demonstrates that anti-CD28 is an improved method for the propagation of these cells in some patients, with a 20-fold greater yield of cells. Immunophenotype analysis also reveals that CD4 T cells are expanded in "TIL" cultures. Furthermore, these cells also exhibited potent cytotoxic activity against DAUDI targets, with 82.8, 69.7, 78.8 and 101.5 percent specific lysis at effector to target ratios of 40:1, 20:1, 10:1 and 5:1.

**TABLE 17**

| **Use of anti-CD28 to Expand TIL cells** | | |
|---|---|---|
| Day of Culture | Tumor cells + IL-2 (1000 U/ml) | Tumor cells + IL-2 anti-CD3, then anti-CD28 |
| 0 | 3.6 X 10⁷ | 3.6 X 10⁷ |
| 5 | 5.8 X 10⁷ | 1.3 X 10⁸ |
| 12 | 1.7 X 10⁸ | 1.4 X 10⁸ |
| 16 | 2.0 X 10⁸ | 2.6 X 10⁸ |
| 19 | 3.8 X 10⁸ | 4.8 X 10⁸ |
| 25 | 2.9 X 10⁸ | 9.0 X 10⁸ |
| 36 | 3.7 X 10⁸ | 1.8 X 10⁹ |
| 43 | 3.0 X 10⁸ | 3.2 X 10⁹ |
| 48 | 2.3 X 10⁸ | 5.1 X 10⁹ |

***Anti-CD3 and anti-CD28 costimulation enhances expression of mRNA for IL-2 and TNF-α in CD4***^{***+***} ***cells.*** To examine whether CD4 cells propagated *in vitro* by anti-CD28 might be an effective source of lymphokines, resting CD4 cells were stimulated by anti-CD3 mAb for 48 h followed by the addition of 50 IU/ml of recombinant IL-2 and compared with CD4 T cells costimulated with anti-CD3 and anti-CD28. Total RNA was harvested from each combination. A total of 10 µg of RNA was loaded into each lane. A class I probe (HLA B7) was used to show uniform loading. The blot was hybridized with ³²P labeled probes specific for IL-2, TNF-α and HLA in succession. On days 1 and 8, the cultures were restimulated with anti-CD3 mAb in the presence of IL-2 or anti-CD28 mAb 9.3 (0.1 µg/ml). The blots were stripped, and rehybridized with a probe for a constant region of HLA class I mRNA, to demonstrate equal loading of the lanes. As illustrated by the Northern Blot of Figure 19, there was a clear enhancement of mRNA for IL-2 and TNF-α after costimulation with anti-CD3 and anti-CD28 and the IL-2 and TNF-α mRNA exceeded that of the IL-2 propagated cells by 10 to 50-fold. Similar results were obtained when the culture was examined for one month of culture, after weekly restimulation with anti-CD3 and IL-2 or anti-CD28.

***Supematants from long-term anti-CD3 and anti-CD28 stimulated cultures of CD4***^{***+***} ***cells contain substantial amounts of IL-2, GM-CSF, and TNF-α*.** After anti-CD3 stimulation in the presence of 200 IU/ml of IL-2 or the combination of anti-CD3 and anti-CD28 in the absence of IL-2, supernatants were tested for IL-2 content using the CTLL-2 cell line, GM-CSF content by ELISA, and TNF-α content by EUSA. The CD4⁺ cells were restimulated with anti-CD3 and IL-2 or anti-CD3 and anti-CD28 respectively at approximately weekly intervals. Anti-CD3 and anti-CD28 cultures of CD4⁺ cells produced roughly the same amount of IL-2 found in supernatant obtained from anti-CD3 activated cells grown in exogenous IL-2. The amount of GM-CSF produced by anti-CD3 and anti-CD28 stimulated CD4⁺ cells was also substantial. Although there were variations in levels of TNF-α depending on when the supernatants were tested, costimulation with anti-CD3 and anti-CD28 was superior to stimulation with anti-CD3 and IL-2 for inducing mRNA for TNF-α. These data indicate that anti-CD28 costimulation with anti-CD3 may not only replace some of the functions of IL-2 but may enhance other synthetic functions of CD4⁺ cells.

### SPECIFIC EXAMPLE XV

### CD28 and CTLA-4 Expression Studies.

***CTLA-4 expression limited to CD28***^{***+***} ***T cells.*** Utilizing site-specific primers and DNA PCR of human genomic DNA, a 348 bp fragment corresponding to exon II of human CTLA-4 was generated, gel purified and used as a ³²P-labeled probe. Purified human T cells were separated into CD28⁺ and CD28⁻ fractions by negative selection with magnetic bead immunoabsorbtion. CD28⁻ T cells were either tested in media or stimulated with PMA + ionomycin or PMA + anti-CD28 mAb (mAb 9.3) for 12 h. CD28⁺ cells were stimulated with the last two conditions for 12 h. RNA was extracted by guanidinium isothicyanate and purified over cesium chloride gradients. Equal amounts of RNA (as determined by ethidium bromide staining) were loaded and separated on a formaldehyde-agarose gel and transferred to nitrocellulose to demonstrate equal loading of RNA. This blot was subsequently probed with the CTLA-4 probe generated above. CTLA-4 was expressed in CD28⁺ cells following PMA or PMA + mAb 9.3 stimulation but not expressed in resting or stimulated CD28-cells. The same blot was hybridized to a HLA probe to confirm equal loading of RNA.

***The expression of CTLA-4 induced under conditions causing CD28 pathway activation.*** Purified resting CD28⁺ T cells were stimulated with PMA alone, ionomycin alone, and PMA + ionomycin for 1 h, 6 h, 12 h and 24 h. RNA was extracted and analyzed by hybridization to CD28 and CTLA-4 probes. Northern blot analysis showed that CTLA-4 expression was induced by PMA or PMA + ionomycin, conditions which are costimulatory with CD28 pathway activation. CTLA-4 expression was not induced by ionomycin, which is not costimulatory with CD28 pathway activation. In contrast, CD28 expression was constant with ionomycin or PMA and even appeared to be suppressed with PMA and ionomycin stimulation. It should also be noted that the induction of CTLA-4 expression occurred as soon as 1 h after stimulation, compared to 6 - 12 h with IL-2 expression following CD28 pathway activation. Since expression of CTLA-4 precedes the biological events caused by CD28 pathway activation (i.e. enhanced IL-2 expression), CTLA-4 expression likely plays a role in the generation of later events.

Purified human T cells were either untreated or stimulated for 1 h, 4 h or 23 h with PMA + PHA, anti-CD28 mAb crosslinked with a second antibody (goat anti-mouse Ig), or anti-CD5 mAb crosslinked in the same manner. Northern blot analysis showed that crosslinking of CD28 receptors, which also can activate the CD28 pathway through mechanisms distinct from PMA and ionomycin, also induced CTLA-4 expression.

***CD28***^{***+***} ***cell lines slightly or not responsive to CD28 pathway activation do not express CTLA-4*.** Two cell lines that are CD28⁺ but responded poorly (T cell line Jurkat CJ) or not at all (myeloma cell line RPMI-8226) to CD28 costimulation as discussed in Kozbor, D. et al., *J*. *Immunol*., 138:4128-4132 (1987) and Ledbetter, J.A. et al., *PNAS (USA),* 84:1384-1388 (1987), were stimulated with PMA + ionomycin + mAb 9.3 and subsequently analyzed by Northern blot for CD28 and CTLA-4 expression. Northern blot analysis of the T cell leukemia cell line Jurkat CJ and of the myeloma cell line RPMI 8226 showed that these cell lines did not express CTLA-4 despite CD28 expression.

T cells were. incubated with various combinations of mitogens including phytohernagglutinin (PHA), phorbol myristate acetate (PMA), and ionomycin (IONO), or anti-CD28 monoclonal antibodies (α-CD28), and examined for the ability to induce CTLA-4 mRNA expression. As shown in Figure 20, the combination of the mitogens PHA and PMA readily induce CTLA-4 expression in normal human T cells, but all combinations tested failed to induce the expression of the CD28-isoform CTLA-4 in the Jurkat T cell line. Both cell types express significant levels of CD28 mRNA under all conditions tested.

Resting T cells were costimulated with anti-CD28 monoclonal antibodies to examine activation in normal human cells. As shown in Figure 21, normal human T cells stimulated by plate-adherent anti-CD3 (α-CD3) monoclonal antibodies at a concentration of 1 µg/ml induced only low levels of CTLA-4 mRNA expression first observable at 1 h after stimulation. In contrast, costimulation of cells with anti-CD3 monoclonal antibodies (1 µg/ml) soluble anti-CD28 (α-CD28) monoclonal antibody (1 µg/ml) led to a dramatic increase in the induction of CTLA-4 mRNA expression.

It should be appreciated that a latitude of modification, change or substitution is intended in the foregoing disclosure and, accordingly, it is appropriate that the appended claims be construed broadly and in a manner consistent with the spirit and scope of the invention herein.

## Claims

1. Use of CTLA4Ig in the manufacture of a medicament for use in down-regulation or suppression of the immune response for the treatment of autoimmune disease or for the treatment of patients receiving an organ transplant.

2. Use according to claim 1 wherein the medicament is for use with an additional immunosuppressant.

3. Use according to claim 2 wherein the immunosuppressant is cyclosporine.

4. Use according to claim 1, 2 or 3, wherein the medicament is for use in reducing rejection of or inducing tolerance to the transplant.

5. Use according to any one of claims 1 to 4 wherein the organ transplant is a heart, lung, kidney, pancreas or liver transplant.

6. Use according to claim 1, 2 or 3 wherein the autoimmune disease is rheumatoid or lyme arthritis.

## Patentansprüche

1. Verwendung von CTLA4Ig für die Herstellung eines Arzneimittels zur Verwendung in der Herunterregulierung oder Unterdrückung der Immunantwort zur Behandlung einer Autoimmunkrankheit oder zur Behandlung von Patienten, die ein Organtransplantat erhalten.

2. Verwendung nach Anspruch 1, wobei das Arzneimittel zur Verwendung mit einem zusätzlichen Immunsuppressivum ist.

3. Verwendung nach Anspruch 2, wobei das Immunsuppressivum Cyclosporin ist.

4. Verwendung nach einem der Ansprüche 1, 2 oder 3, wobei das Arzneimittel zur Verwendung zur Verminderung der Abstoßungsreaktion oder Induktion der Toleranz gegenüber dem Transplantat ist.

5. Verwendung nach einem der Ansprüche 1 bis 4, wobei das Organtransplantat ein Herz-, Lungen-, Nieren-, Bauchspeicheldrüsen- oder Lebertransplantat ist.

6. Verwendung nach einem der Ansprüche 1, 2 oder 3, wobei die Autoimmunkrankheit rheumatoide Arthritis oder Lyme-Krankheit ist.

## Revendications

1. Utilisation d'une immunoglobuline CTLA4 dans la fabrication d'un médicament pour son utilisation dans la suppression ou la régulation négative de la de la réponse immunitaire pour le traitement d'une maladie auto-immune ou pour le traitement de patients ayant reçu une greffe d'organe.

2. Utilisation selon la revendication 1, dans laquelle le médicament est destiné à être utilisé avec un immunosuppresseur.

3. Utilisation selon la revendication 2, dans laquelle l'immunosuppresseur est la cyclosporine.

4. Utilisation selon l'une quelconque des revendications 1 à 3, dans laquelle le médicament est utilisé pour réduire le rejet ou pour augmenter la tolérance à la greffe.

5. Utilisation selon l'une quelconque des revendications 1 à 4, dans laquelle l'organe greffé est un coeur, un poumon, un rein, un pancréas ou un foie.

6. Utilisation selon l'une quelconque des revendications 1 à 3, dans laquelle la maladie auto-immune est l'arthrite rhumatoïde ou l'arthrite de Lyme.
